(19) Europäisches Patentamt
European Patent Office
Office européen des brevets

(11) EP 3 918 320 B1

(12) **FASCICULE DE BREVET EUROPEEN**

(45) Date de publication et mention de la délivrance du brevet:
**02.10.2024 Bulletin 2024/40**

(21) Numéro de dépôt: **19832420.4**

(22) Date de dépôt: **31.12.2019**

(51) Classification Internationale des Brevets (IPC):
**G01N 33/02** (2006.01)    **A01K 11/00** (2006.01)

(52) Classification Coopérative des Brevets (CPC):
**G01N 33/02; A01K 11/00**

(86) Numéro de dépôt international:
**PCT/EP2019/087197**

(87) Numéro de publication internationale:
**WO 2020/141179 (09.07.2020 Gazette 2020/28)**

(54) **MARQUAGE ET IDENTIFICATION ISOTOPIQUES DE LIQUIDES**

ISOTOPENMARKIERUNG UND IDENTIFIZIERUNG VON FLÜSSIGKEITEN

ISOTOPIC MARKING AND IDENTIFICATION OF LIQUIDS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **02.01.2019 FR 1900017**

(43) Date de publication de la demande:
**08.12.2021 Bulletin 2021/49**

(73) Titulaire: **IDS Group**
**69330 Meyzieu (FR)**

(72) Inventeurs:
• **FAUVET, Patrice**
  **69006 LYON (FR)**
• **MIGEON, Valérie**
  **69003 LYON (FR)**

(74) Mandataire: **Lavoix**
  **62, rue de Bonnel**
  **69448 Lyon Cedex 03 (FR)**

(56) Documents cités:
**DE-A1- 10 200 802    FR-A1- 3 068 135**
**US-A- 5 760 394    US-A1- 2012 323 809**

**Description**

La présente invention concerne le marquage des liquides et des sous-produits issus d'une transformation (vins, spiritueux, pétillants, champagne, jus, sirop, concentré, eau, gélatine, lait et produits dérivés du lait). Elle concerne notamment une méthode d'identification isotopique d'un liquide et de ses sous-produits, ainsi qu'une méthode permettant d'imposer un code d'identification unique et permanent à ces liquides, et à ses sous-produits. L'invention concerne enfin un support d'enregistrement d'information et un calculateur électronique pour la mise en oeuvre d'un tel procédé.

La possibilité de déterminer l'origine des liquides et ses sous-produits est devenue un enjeu de sécurité alimentaire et un facteur de pénétration des marchés par les viticulteurs, agriculteurs et transformateurs du secteur. Les syndicats viticoles et agricoles, les coopératives, les négociants en vins, ainsi que la grande distribution sont également de plus en plus concernés par les problématiques de fraude à l'appellation, de contrefaçon et des problèmes de santé alimentaire.

Il est connu de l'US 2012/323809 A1 d'encoder l'origine des liquides en modifiant le ratio naturel des isotopes des éléments C, N, O, H, et Ca.

La présente invention a pour objectif de proposer une méthode permettant d'imposer de manière sûre et reproductible un code unique à un liquide et à ses sous-produits, permettant de connaître son origine, et cela de préférence avec une précision allant jusqu'au lieu de culture du végétal dont il est issu (exemples les jus de fruits dont le raisin), du lieu d'élevage des animaux dont il est issu (exemple le lait), du lieu de collecte (exemple des eaux minérales et du lait), de production ou de transformation (y compris vinification).

Elle a notamment pour objectif de fournir un code unique affecté permettant une traçabilité à des niveaux de granularité différents, par exemple d'un domaine, d'une parcelle ou d'une appellation, ou beaucoup plus fins, tels qu'une cuve et, optionnellement, la datation du millésime, ceci permettant aussi d'identifier une fraude éventuelle.

Un autre objectif de l'invention est de proposer une méthode d'identification sûre et reproductible d'un liquide et de ses sous-produits, permettant de connaître son origine, et cela de préférence avec une précision allant au moins jusqu'au lieu de culture, d'élevage, de production ou de transformation.

Un autre objectif de l'invention est de proposer une méthode d'identification permettant de caractériser une fraude sur un produit, comme la dilution, le coupage, le mélangeage, actes qui conduisent à un produit qui n'est pas conforme à ce qu'il devrait être.

Un autre objectif encore de l'invention est de fournir de telles méthodes adaptées à la gestion de plusieurs ou de nombreux domaines ou de lieux de transformation, et de préférence sans limitation dans le nombre de domaines et de lieux de transformation.

Un autre objectif est de fournir de telles méthodes qui soient économiquement viables.

Résumé de l'invention

Ces objectifs ainsi que d'autres, sont atteints notamment grâce à l'utilisation d'un modèle prédéfini (M) employé pour imposer aux liquides et leurs sous-produits un code isotopique unique propre à un domaine (e.g. domaine viticole, agricole ou d'élevage laitier), une usine de transformation (e.g. produits dérivés du lait), et éventuellement à des niveaux de granularité encore plus fins (e.g. cuves, éventuellement le millésime), ce code étant basé sur la nature, les concentrations d'éléments chimiques ou les concentrations ou les ratios en isotopes stables d'éléments chimiques. Ces modèles peuvent être améliorés par la mesure, la connaissance et la prise en compte des concentrations en éléments et/ou en isotopes d'éléments majeurs apportés par la nature (les sols, l'eau, l'incorporation par les végétaux et animaux) mais également par un processus opéré par l'homme, et pouvant apporter un élément de signature prévisible et analysable dans les liquides.

Au fur et à mesure du déploiement de la solution à plusieurs domaines ou lieux/usines de transformation, des codes uniques peuvent être générés pour chacun d'entre eux, en tenant compte des codes précédemment générés pour d'autres domaines ou lieux/usines de transformation, éventuellement en plus avec une notion de cycle de production du même domaine ou du même lieu/usine de transformation. Ces codes uniques sont générés de préférence par type de produit. Comme on le comprendra, on peut aussi raisonner en termes de génération de codes uniques pour des produits, dont on pourra ensuite contrôler l'authenticité et éventuellement détecter une altération frauduleuse ou une manipulation de l'origine.

Tous ces codes peuvent être enregistrés dans le modèle (M), de préférence logé dans un calculateur électronique ou similaire. Chaque code unique correspond à la signature élémentaire et isotopique du liquide tel qu'il doit être retrouvé jusqu'à la fin de sa consommation. Ce code peut être atteint dans le produit au moment de sa production, par exemple au moment de la vinification, ou de la transformation du produit et de ses sous-produits. En cas de transformation, on retient avantageusement la signature élémentaire et isotopique du dernier stade du produit à partir duquel il n'y a plus de variation sensible de la composition de la matière sèche, des éléments chimiques et des concentrations ou des ratios d'isotopes stables, sinon il convient d'appliquer un correctif, comme il sera expliqué plus loin. Une fois le code établi et imposé au produit, ce code ne varie plus après transformation (par exemple après vinification, traitement du lait et des produits dérivés), sauf cas d'application d'un correctif, et marque donc également les produits issus des liquides et des sous-produits ainsi marqués, donc les produits finis (sans impact significatif d'un éventuel processus de vieillissement pour le vin par exemple) et les produits emballés.

L'effet d'accumulation des marqueurs (isotopes ajoutés) est terminé une fois l'intégration effectuée dans le processus. L'invention tient donc compte des points importants qu'est le moment du cycle de marquage (c'est-à-dire de l'intégration isotopée des marqueurs). On peut aussi tenir compte de la durée requise pour l'homogénéisation des marqueurs dans un liquide. Cette homogénéisation peut faire appel à un instrument (injecteur, mélangeur, etc.) pour accélérer la répartition, le mélange, l'homogénéisation des marqueurs dans un volume de production et de sa durée.

Ce code est le résultat d'une intégration isotopée déterminée par le modèle (M) et appliquée par le responsable de site (par exemple maître de Chais ou responsable du processus) selon les directives données par le modèle. Pour aboutir à ce code unique lors de la production, de la vinification ou de la transformation d'un liquide, le modèle (M) peut tenir compte du taux d'intégration ou d'accumulation (TA) des éléments et/ou isotopes dans le liquide en fonction du processus d'ajout des isotopes et de transformation du produit.

Le moment d'intégration dans le processus peut être un facteur de sécurité supplémentaire. Cette intégration peut par exemple être apportée en une ou plusieurs fois ou sur une seule période ou plusieurs périodes, par exemple 2 ou 3. Elle peut être réalisée juste au démarrage de la vinification, avant la mise en bouteille ou dans le cadre d'un processus de transformation. A titre d'exemple, pour le vin, l'intégration isotopée peut être effectuée le plus en amont possible du processus de vinification afin de garantir tout risque de fraude à l'appellation (par exemple par des coupages avec d'autres vins à un stade ultérieur, des dilutions pour les spiritueux). Ce code peut aussi intégrer les marqueurs ou éléments dits majeurs qui ont une variabilité selon les cycles, les lots, les appellations ou les millésimes en fonction de la nature du liquide. Les paramètres peuvent avoir été déterminés au préalable par essais, par exemple sur un ensemble de cuve dans le cas du vin.

L'invention prévoit la possibilité de disposer de plus d'un code unique ou de plusieurs fractions identifiables et exploitables d'un code unique dans le produit final, permettant de remonter à plus d'information ou notamment à des intervenants successifs dans la chaine de production.

Dans un mode de réalisation, l'incorporation à plusieurs stades (par exemple 2) permet d'obtenir plusieurs signatures (par exemple 2). De préférence, chaque incorporation permet d'imposer un code spécifique sans que la ou les incorporations précédente(s) ou ultérieure(s) ne le modifie de manière substantielle (c'est-à-dire sans nuire à la viabilité et à la reconnaissance du code). Ce mode de réalisation permet notamment de disposer d'un code précoce, qui pourra être retrouvé en cas de mélange ou de vente d'un liquide à un tiers. Ainsi, un code unique spécifique peut être imposé en premier lieu, notamment par un premier producteur, puis au moins un deuxième code unique spécifique est ensuite imposé, par le même producteur ou un producteur ultérieur. Chacun de ces deux codes pourra être retrouvé grâce à l'invention. Pour prendre des exemples, un viticulteur, puis un distilleur ; un premier viticulteur, puis un assembleur ; un récoltant, puis un transformateur.

De manière préférée et avantageuse, le choix des éléments et de leurs isotopes, et de leurs ratios respectifs que le Modèle (M) détermine pour chaque domaine ou sous-ensemble (parcelles), ou pour entité (lieu, usine, etc.) de transformation, est basé sur la connaissance préalable de ce qu'on appelle ici la signature géochimique basale (SGB) d'une récolte (vendange ou sous-ensemble (parcelles), ou d'une entité de transformation. Cette SGB, comme il sera décrit plus loin, est la connaissance d'éléments chimiques, d'isotopes stables de certains éléments, de leurs concentrations et/ou ratios respectifs, au sein d'un domaine ou de son sous-ensemble, ou d'une entité de transformation, préalablement à l'imposition du code par l'intégration d'une solution isotopée. Le modèle (M) peut avantageusement intégrer la capacité à définir une solution isotopée permettant d'imposer ledit code unique, en faisant varier les isotopes stables d'éléments présents dans la SGB ainsi que leurs concentrations ou leurs ratios. De préférence, le modèle (M) va faire cela de la manière la plus minimaliste qui soit et en tenant compte du prix /mode de production des isotopes/niveau d'enrichissement/stratégie des isotopes en fonction des marchés ou de la disponibilité des isotopes afin que le prix de l'intégration d'isotopes soit le plus bas possible. Toutes ces caractéristiques s'appliquent aux objets de l'invention définis plus en détail ci-après. Ce modèle (M) peut permettre en outre, le cas échéant, de relier un liquide (par exemple vin, champagne, spiritueux ou du lait) à un domaine ou un produit issu d'une entité de transformation déterminée, par analyse de concentrations ou de ratios d'éléments et d'isotopes stables, permettant de déterminer un profil de concentrations ou de ratios d'éléments et d'isotopes stables, notamment par spectrométrie de masse, et de comparer aux codes uniques enregistrés dans le modèle (M). Certains éléments et leur répartition isotopique (concentrations ou ratios des éléments suivants ou de leurs isotopes stables : C, H, O, N, S) varient au cours de l'année, par exemple en fonction des apports du sol, de la nourriture ou de l'eau. L'invention permet aussi de déterminer le cycle de production d'un liquide ou d'un sous-produit, à partir d'un échantillon de celui-ci.

Au sens de l'invention, on entend, notamment, par sous-produit, tout ce qui est issu d'un liquide naturel ou transformé. Il peut notamment s'agir de produits dérivés (par exemple le vinaigre, des huiles ou des produits semi liquides tels que les produits laitiers dont les yaourts, les fromages, les crèmes ou les beurres). La méthode selon l'invention peut être appliquée sur un liquide destiné à la consommation des êtres humains, mais pas seulement.

Ainsi, l'invention a-t-elle pour objets notamment une méthode d'identification isotopique et une méthode permettant d'imposer un code unique, ainsi qu'un calculateur électronique.

L'invention concerne plus particulièrement la méthode permettant d'imposer un code propre à des liquides, de préférence vin ou spiritueux, d'un lieu d'origine ou à des sous-produits de ce liquide,

- méthode dans laquelle on dispose de la signature géographique basale (SGB) du liquide ou de son sous-produit, comprenant :

  (i) les concentrations d'au moins 5, 6, 7, 8, 9, 10, 11, 12 ou la totalité des éléments du groupe (3) Rb, Sr, B Li, Ca, Na, Mg, K, F, P, Cl, As, Pb, Cd,
  (ii) les concentrations d'au moins 5, 10, 15, 20, 25, 30, ou la totalité des éléments du groupe (2) : Be, Al, V, Cr, Mn, Fe, Co, Ni, Cu, Zn, Ga, Ge, Se, Y, Zr, Nb, Mo, Rh, Pd, Ag, Sn, Sb, Te, I, Ba, Hf, Ta, W, Re, Ir, Hg, Ti, Si, et
  (iii) les concentrations d'isotopes stables d'au moins 1, 2, 3, 4, 5, 6, 7, 8, 9 ou 10 des éléments de ce groupe (2) ;

  - le méthode comprenant l'ajout à ce liquide ayant cette SGB, d'une quantité connue d'au moins 1 isotope stable d'au moins 1, 2 ou 3 éléments chimiques du groupe (2), ce grâce à quoi l'on obtient un liquide ayant une signature isotopique déterminée, avec un ratio d'isotope modifié par rapport à la SGB.

La SGB peut comprendre en outre les concentrations des éléments chimiques d'au moins 2, 3, 4 ou les 5 éléments chimiques du groupe (1) : C, O, N, H, S.

La SGB comprend notamment des concentrations d'isotopes stables d'au moins 1, 2 ou 3 des éléments du groupe (2') Al, Mn, Fe, Co, Cu, Zn, Mo, Sn, Ti, Si.

De préférence, on ajoute au liquide une quantité connue d'au moins 1 isotope stable d'au moins 1, 2 ou 3 des éléments du groupe (2') Al, Mn, Fe, Co, Cu, Zn, Mo, Sn, Ti, Si.

La SGB comprend notamment les concentrations d'au moins 1, 2 ou 3 isotopes stables d'au moins 1, 2 ou 3 des éléments Fe, Cu, Zn, Mo, Sn, Ti, Si.

De préférence, on détermine pour la SGB les concentrations d'au moins 2 isotopes stables d'au moins 1 élément parmi Fe et Zn. On mesure notamment $^{56}Fe$ et $^{57}Fe$, et/ou $^{66}Zn$ et $^{68}Zn$. De préférence, par ajout d'isotope dans le liquide, on fait varier la concentration de $^{56}Fe$ et/ou de $^{57}Fe$, et/ou de $^{66}Zn$ et/ou de $^{68}Zn$.

La SGB comprend notamment les concentrations des éléments Rb, Sr et B.

La SGB comprend notamment les concentrations des éléments Li, Ca, Na, Mg, K, F, P, Cl, As, Pb, Cd.

Il est possible d'affiner les signature en mesurant, pour les éléments ci-dessus, notamment pour un nombre raisonnable et suffisant d'éléments, les concentrations de certains ou de tous leurs isotopes stables. Il peut s'agir notamment de mesurer ces isotopes stables pour de 1 à 10 éléments en plus de ceux du groupe (2), notamment choisis parmi les majeurs et/ou les éléments du groupe (3). Un nombre est jugé suffisant s'il apporte une discrimination ou une localisation précise pour le type de produit considéré. Le groupe (1) concerne les éléments « majeurs ». La connaissance de ces majeurs et de leurs isotopes stables donne notamment une indication de localisation géographique relativement précise, par exemple régionale. Le groupe (3) constitue les éléments trace majeurs. La connaissance de ces éléments trace majeurs et de leurs isotopes stables, combinée notamment aux majeurs, donne notamment une indication de localisation géographique plus fine, par exemple locale.

Dans un mode de réalisation, le code unique intègre en plus l'apport d'isotopes stables au cours du processus de production. Ceci signifie que le code unique additionne l'effet de l'ajout initial (ou initiaux si on les ajoute en plusieurs fois) d'isotopes au liquide d'origine et l'effet des apports postérieurs d'isotopes, notamment ceux apportés par la transformation du liquide d'origine, par exemple par le bois d'un tonneau pour les vins et spiritueux. Dans un mode de réalisation le code unique intègre l'effet de l'élimination de matière sèche lors du processus de production. Ces effets ultérieurs peuvent être quantifiés ou modélisés avec suffisamment de précision.

Selon différents modes de réalisation, l'adjonction isotopée peut être ainsi réalisée :

- Pour les spiritueux, juste avant la mise en tonneau ou autre contenant de vieillissement, ou juste avant mise en bouteille ;
- Pour le vin, juste après pressurage et fermentation, notamment après la clarification ou filtration, ou après une période de maturation, ou juste avant mise en bouteille ;
- Pour le lait, marquage de la cuve de collecte propre à une exploitation ou commune à plusieurs exploitations agricoles ;
- Pour le Champagne, marquage dans la Liqueur ;
- Pour les sirops, marquage dans la cuve après pressage et collecte du concentré ;
- Pour les produits transformés, lors du mélange initial de l'ensemble des ingrédients. Ce principe est préféré pour tout produit manufacturé.

Dans un mode de réalisation le code unique est défini par un calculateur électronique en fonction des codes uniques déjà générés et enregistrés et de la SGB du liquide ou de son sous-produit, ce calculateur électronique comprenant un modèle (M) contenant en mémoire les codes uniques déjà générés.

Dans un mode de réalisation on mesure la SGB de liquides ou de sous-produits successifs de même type et l'on définit à chaque fois un code unique au liquide ou sous-produit dernièrement analysé, code qui se distingue des codes uniques précédemment définis et enregistrés dans le modèle (M).

L'invention a notamment pour objet une méthode d'identification isotopique permettant de relier un liquide ou un sous-produit, de préférence vin ou spiritueux, à un lieu d'origine, la méthode comprenant :

   a- dans un échantillon du liquide ou du sous-produit, la mesure :

   (i) des concentrations d'au moins 5, 6, 7, 8, 9, 10, 11, 12 ou la totalité des éléments du groupe (3) Rb, Sr, B Li, Ca, Na, Mg, K, F, P, Cl, As, Pb, Cd,
   (ii) des concentrations d'au moins 5, 10, 15, 20, 25, 30, ou la totalité des éléments du groupe (2) : Be, Al, V, Cr, Mn, Fe, Co, Ni, Cu, Zn, Ga, Ge, Se, Y, Zr, Nb, Mo, Rh, Pd, Ag, Sn, Sb, Te, I, Ba, Hf, Ta, W, Re, Ir, Hg, Ti, Si, et
   (iii) les concentrations d'isotopes stables d'au moins 1, 2, 3, 4, 5, 6, 7, 8, 9 ou 10 des éléments de ce groupe (2) ;

   obtention d'un profil de concentrations d'éléments et d'isotopes stables;
   b- la comparaison de ce profil avec des profils enregistrés dans un modèle prédéfini (M) contenant en mémoire des profils sous forme de codes uniques chacun propre à un type de liquide ou sous-produit d'un lieu d'origine, chaque code unique ayant été préalablement généré par le modèle (M) avec une variation d'isotopes d'éléments,
   c- la conclusion que le liquide ou le sous-produit dérivé à identifier a un profil sensiblement égal à un code enregistré et donc indication d'un lieu d'origine, si, à l'issue de la comparaison, le profil correspond à un profil enregistré et, dans le cas contraire, la conclusion que le liquide ou le sous-produit dérivé ne provient d'aucun lieu d'origine dont le code est enregistré dans le modèle, ou que le liquide a été falsifié.

De préférence, on mesure en outre dans un échantillon du liquide ou du sous-produit, les concentrations d'au moins 2, 3, 4, ou les 5 éléments chimiques du groupe (1) : C, O, N, H, S.

La SGB comprend notamment des concentrations d'isotopes stables d'au moins 1, 2 ou 3 des éléments du groupe (2') Al, Mn, Fe, Co, Cu, Zn, Mo, Sn, Ti, Si.

De préférence, on ajoute au liquide une quantité connue d'au moins 1 isotope stable d'au moins 1, 2 ou 3 des éléments du groupe (2') Al, Mn, Fe, Co, Cu, Zn, Mo, Sn, Ti, Si.

La SGB comprend notamment les concentrations d'au moins 2 isotopes stables d'au moins 1, 2 ou 3 des éléments Fe, Cu, Zn, Mo, Sn, Ti, Si.

De préférence, on détermine pour la SGB les concentrations d'au moins 2 isotopes stables d'au moins 1 élément parmi Fe et Zn. On mesure notamment 56Fe et 57Fe, et/ou 66Zn et 68Zn. Par ajout d'isotope dans le liquide, on fait varier la concentration de 56Fe et/ou de 57Fe, et/ou de 66Zn et/ou de 68Zn.

La SGB comprend notamment les concentrations des éléments Rb, Sr et B.

La SGB comprend notamment les concentrations des éléments Li, Ca, Na, Mg, K, F, P, Cl, As, Pb, Cd.

D'autres objets encore apparaîtront à la lecture de la description détaillée qui suit.

### Description détaillée

Définitions :

Sauf indication contraire, on regroupera sous l'appellation générique « lieu d'origine» d'un liquide ou sous-produit, notamment :

- le domaine viticole ou agricole produisant le végétal dont le liquide est issu et/ou assurant la production ou la transformation d'un végétal ou d'un liquide : par exemple vinification, élevage, assemblage, vieillissement, pour les vins et spiritueux; par exemple concentration, pressage, extraction, pasteurisation pour les jus de fruit ;
- l'éleveur des animaux dont le liquide est issu (exemple le lait) et/ou le lieu ou l'entreprise de production ou de transformation de produits laitiers : par exemple pasteurisation, stérilisation, concentration, production de yaourts, fromages, etc. ;
- le lieu ou l'exploitation de collecte (exemple des eaux minérales).

Par « sous-ensemble d'un lieu d'origine », on entendra notamment parcelle de vigne, parcelle de verger, ensemble ou sous-ensemble d'animaux d'une ferme, ou beaucoup plus fins, tels qu'une cuve, tonneau, foudre, et tout contenant utilisé dans le domaine des vins et spiritueux, la datation du millésime, etc.

Sauf indication contraire, l'appellation « lieu d'origine », lorsqu'elle sera utilisée, englobera les « sous-ensembles ».

Sauf indication contraire, on regroupera sous l'appellation générique « cycle productif » un cycle de culture, un cycle d'élevage d'animaux, un cycle de production et/ou un cycle de transformation. Dans le domaine particulier du vin ou des spiritueux, ce cycle productif peut correspondre au millésime.

Sauf indication contraire, l'appellation « cycle productif » pour un liquide, lorsqu'elle sera utilisée, englobera les « sous-produits » de ce liquide, donc les produits de transformation, extraction, etc. issus du liquide d'origine. De préférence, les transformations s'entendront avec conservation de la matière sèche et/ou sans apport exogène de matière dont on ne connaît pas l'apport isotopique.

L'objectif de l'invention est d'imposer un code unique à un produit se trouvant dans le commerce ou destiné à s'y retrouver. Ce produit peut être un « liquide d'origine » non ou peu transformé, comme un jus de raisin ou du lait. Le plus souvent, il s'agit d'un « produit transformé », par exemple le vin, le lait dont on a ajusté la teneur en matière grasse et/ou que l'on a pasteurisé, stérilisé (UHT), ou transformé en fromages, yaourts, et autres produits laitiers. En conséquence, le code unique est celui présent dans ce produit transformé lorsqu'il n'a pas été altéré ou falsifié et se retrouve sur le marché.

Par « ratios d'isotopes présents de manière stable » dans un liquide d'origine, on entend les ratios d'isotopes d'éléments chimiques du liquide d'origine au stade où ces ratios ne varient plus ; en d'autres termes, le liquide d'origine ne voit plus sa composition en isotopes varier (sauf s'il fait l'objet d'une altération ou falsification, ce que la méthode de l'invention permet de démontrer).

Par « ratios naturels d'isotopes », on entend les ratios d'isotopes d'éléments chimiques qui se retrouvent dans les liquides et produits transformés du fait du processus de production comprenant notamment l'élevage, la culture, la récolte ou la collecte, et les transformations, en excluant les apports isotopés volontaires destinés à finaliser le code unique. Pour ces derniers on pourra parler de « ratios d'isotopes imposés ».

**Méthode d'identification isotopique**

La méthode d'identification isotopique permet, le cas échéant, de relier un liquide d'origine à un lieu de récolte ou de transformation déterminé (le lieu d'origine), et/ou encore à caractériser une altération ou falsification d'un produit d'origine, et/ou à démontrer une fraude à l'appellation d'origine, par analyse de concentrations ou de ratios d'isotopes stables.

La méthode peut comprendre notamment, dans un échantillon issu du liquide d'origine à identifier au cours d'une étape a-, la mesure de concentrations (C2) d'éléments chimiques et/ou d'isotopes stables d'éléments chimiques. Il est possible de calculer des ratios (R2) entre éléments ou plus particulièrement entre isotopes stables, notamment d'un même élément ou de plusieurs. Ceci permet l'obtention d'un profil de concentrations ou de ratios de ces éléments et isotopes stables, notamment par spectrométrie de masse.

Elle peut aussi comprendre au cours d'une étape b-, la comparaison de ce profil avec des profils enregistrés dans un modèle prédéfini (M) contenant en mémoire des profils sous forme de codes uniques chacun propre à lieu de récolte ou un lieu de transformation, chaque code unique ayant été préalablement généré par le modèle (M) et appliqué de manière unique à un lieu d'origine par adjonction de matière isotopée à ces liquides de manière que, au moment de leur /transformation, les concentrations ou ratios de ces isotopes stables dans dérives soient sensiblement identiques au code unique,

Elle peut aussi comprendre au cours d'une étape c-, la conclusion que le liquide ou le produit issu d'un liquide à identifier a un profil sensiblement égal à un code enregistré (un intervalle [min, maxi]) et en outre indication du lieu de récolte ou de transformation ou un sous-ensemble de ce lieu prenant part à la transformation de ce liquide, si, à l'issue de la comparaison, le profil correspond à un profil enregistré et, dans le cas contraire, la conclusion que le liquide ou le produit ne provient d'aucun lieu de récolte/transformation ou sous-ensemble du lieu récolte/transformation dont le code est enregistré dans le modèle.

On parle de profil sensiblement égal à un code enregistré. On tient compte d'une certaine variabilité de la signature au moment de l'analyse et du degré de précision des appareils de mesure. Pour tenir compte de cette variabilité, les codes uniques seront de préférence générés en tenant compte de cette variabilité comme une donnée d'entrée afin d'éviter les chevauchements possibles lors de la mesure entre des profils enregistrés/codes uniques. On retiendra notamment un intervalle de 0,0001 à 10%, de préférence à 5, 4, 3, 2, 1, ou 0,1 %.

Dans ce qui suit, le terme sous-ensemble d'un lieu de récolte/transformation ne sera pas toujours repris en même temps que le terme lieu de récolte/transformation, mais on considère qu'il est compris dans le terme lieu de récolte/transformation.

Dans ce qui suit, les mesures de concentrations (C2) ou de ratios (R2) d'éléments ou d'isotopes stables d'un ou plusieurs éléments permettent d'obtenir des profils isotopiques en ratios d'isotopes d'un même élément et aussi, de préférence, des ratios entre éléments chimiques. Les mesures sont réalisées par les méthodes de mesure fiables pour les éléments considérés (voir plus loin).

Notamment, à l'étape a- d'une méthode hors du cadre de l'invention, on mesure la concentration des éléments chimiques suivants :

- au moins 3, 4, 5, 10, 15, 20, 25, 30, ou la totalité des éléments du groupe (2) : Be, Al, Ti, V, Cr, Mn, Fe, Co, Ni, Cu, Zn, Ga, Ge, Se, Rb, Y, Zr, Nb, Mo, Rh, Pd, Ag, Sn, Sb, Te, I, Ba, Hf, Ta, W, Re, Ir, Hg, Ti, Si, et/ou au moins 3, 4, 5, 10, ou la totalité des éléments du groupe (3) : Sr, B, Li, Ca, Na, Mg, K, F, P, Cl, As, Pb, Cd ; et/ou
- au moins 3, 4, 5, 8, 12, ou la totalité des éléments suivants : du groupe (4) La, Ce, Pr, Nd, Pm, Sm, Eu, Gd, Tb, Dy, Ho, Er, Tm, Yb, Lu et/ou, de préférence et,
- au moins 2, 3, 4 ou la totalité des éléments du groupe (1) C, O, N, H, S.

De préférence, le modèle prédéfini (M) comprend les codes uniques d'autres lieux d'origine pour des produits similaires et/ou les codes uniques attribués à un lieu d'origine donné par rapport auquel on peut vouloir vérifier l'authenticité d'un produit ou sa falsification. Ce modèle (M) est donc renseigné avec les codes uniques, donc les ratios ou concentrations d'un certain nombre d'éléments chimiques ou d'isotopes d'un certain nombre d'éléments chimiques. Bien entendu, il faut une corrélation suffisante entre les éléments chimiques définissant les codes uniques enregistrés dans le modèle et les éléments chimiques recherchés dans l'échantillon à comparer. Pour chaque type de produit et/ou son origine, il est possible de présélectionner les groupes d'éléments représentatifs dont on mesurera les ratios d'isotopes tant pour construire les codes uniques que pour effectuer les analyses ultérieures.

Nous verrons plus loin des modalités préférées de gestion des éléments et de leurs ratios, combinant des mesures de certains d'entre eux, de préférence au niveau de chaque cycle de récolte ou de transformation, et des variations de certains autres par les liquides ou sous-produits dérivés.

De préférence, ce code unique pour un lieu de récolte ou de transformation a été déterminé par le modèle prédéfini (M), et imposé aux liquides naturels ou transformés ou tous produits dérivés par l'adjonction isotopée distribuée de manière que ce code soit intégré par le liquide ou son sous-produit, d'une manière permanente ou d'évolution connue. De cette manière, tous les liquides et les liquides transformés ou sous-produits ayant ce code unique et le modèle M a en mémoire l'ensemble des codes uniques générés à un temps donné.

De préférence, l'étape a- comprend, pour chaque élément chimique pour lequel on s'intéresse aux isotopes stables, la détermination des variations d'un ou plusieurs isotopes stables mineurs par rapport au plus abondant.

De préférence, les concentrations ou ratios d'isotopes sont mesurées par spectrométrie de masse (SM). On peut notamment utiliser la spectrométrie de masse à plasma à couplage inductif, ou ICP-MS, tout particulièrement la spectrométrie de masse à source plasma et à multi-collection (MC-ICPMS), ou la spectrométrie de masse à ratio isotopique, ou IRMS, ou toute autre technique permettant de mesurer les concentrations des éléments chimiques recherchés, et/ou d'identifier et mesurer les éléments et leurs isotopes stables, avec leurs concentrations ou leurs ratios, par exemple exprimé en ratios du ou des isotopes stables mineurs par rapport à l'isotope stable le plus abondant dans l'échantillon. Il est ainsi également possible de procéder à des analyses de concentration par spectrométrie de masse à plasma induit couplée à l'ablation laser (LA-ICP/MS, en anglais Laser Ablation Inductively Coupled Plasma Mass Spectrometry) ou par spectroscopie sur plasma induit par laser (LIBS, en anglais Laser Induced Breakdown Spectroscopy).

La méthode peut notamment comprendre l'utilisation d'un calculateur électronique programmable, pourvu d'une unité logique programmable, d'un support d'enregistrement d'informations et d'une interface d'échange de données raccordés entre eux par un bus de données interne. Le calculateur électronique peut également comporter une interface homme-machine.

La méthode peut donc comprendre l'acquisition, par le calculateur électronique, des valeurs de concentrations (C2) et/ou ratios (R2), notamment telles que mesurées par SM et/ou autre méthode, formant la signature à composante isotopique d'un liquide ou d'un sous-produit issu de ce liquide, dont on veut savoir s'il provient d'un lieu de récolte ou de transformation, d'un sous-ensemble d'un lieu de récolte ou unité de transformation dont le code unique est connu ou enregistré dans le calculateur électronique, et, le cas échéant, de connaître l'origine exacte et/ou sa conformité ou sa falsification.

Le calculateur peut donc aussi effectuer la comparaison entre les données acquises par le calculateur et les codes uniques enregistrés qu'il a en mémoire. Le calculateur peut ensuite déterminer par cette comparaison si le profil acquis correspond ou pas à un code unique enregistré et délivrer dans le premier cas l'identité du lieu d'origine ou alors conclure qu'il n'y a aucune correspondance ou encore une falsification.

Il est en effet tout à fait possible d'identifier une falsification en comparant l'étiquetage d'un produit dont on connaît la signature qui devrait être retrouvée et la signature à composante isotopique mesurée sur le produit contrôlé.

On peut aller plus loin, par exemple on peut très bien connaître des marqueurs isotopiques propres par exemple à une appellation d'origine, et il est alors possible, de vérifier par l'analyse de ces marqueurs, si le produit contrôlé a bien l'origine qu'il prétend avoir.

Exemple sur le Marquage des spiritueux contre la fraude : Les spiritueux tel que les bouteilles de cognac font régulièrement l'objet de fraude par la dilution en utilisant de l'eau, un alcool agricole ou d'un équivalent neutre. Ceci altère la composition du produit et la plupart du temps, le consommateur final ne le remarque pas et paye au prix le plus fort. La méthode anti-fraude consiste à utiliser une solution dopée en isotope permettant de marquer, puis d'identifier par analyse une appellation, un domaine ou une cuvée spécifique et de vérifier les éventuelles dilutions qui pourraient être effectuées frauduleusement. Grâce à la solution isotopique (Code unique) connue en termes de composition élémentaire et de sa concentration, il est possible d'estimer la dilution effectuée dans une bouteille de spiritueux en comparant la recette initiale du code unique et un échantillon issu d'un même lot de bouteilles marquées.

Autre exemple : Les vins IGP sont coupés/mélangés avec des vins de provenance d'un autre pays. L'exemple le plus récent étant la fraude aux vins rosés dans le sud de la France. Les vins sont achetés en Espagne et revendus en France sans tenir compte de leur origine. Le consommateur est dupé et paye pour une qualité inférieure à celle attendue.

Marquage anti-contrefaçon : Les grands crus ou vins de qualité font l'objet de Contrefaçon en raison de leur prix élevé. Des revendeurs proposent des vins qui ne correspondent au domaine d'origine tout s'étant assurés de bien copier l'étiquette du Domaine. Le marquage isotopique du vin à l'échelle d'un domaine permet d'identifier de manière irrévocable que le vin est du domaine d'origine ou non. La recette du code unique initialement intégrée dans la cuve de vinification (avantageusement, le marquage est effectué le plus en amont de la vinification, en général juste après les vendanges afin d'éviter les risques également de fraude). Lors d'un contrôle via un échantillon d'une bouteille de vin (via l'utilisation d'un cornavin, appareil permettant d'effectuer un prélèvement dans une bouteille sans débouchonner), l'analyse va permettre de révéler la composition isotopique et sa teneur faisant référence à un code unique désignant un lieu ou un domaine la base de données des codes uniques.

Marquage pour éviter la fraude au millésime : Certains domaines notamment les champagnes, produisent pour certaines années des cuvées millésimées. Cependant quand une cuvée a moins de succès, certains domaines n'hésitent pas à changer l'année en faisant référence à un millésime ayant obtenu un certain succès afin de vendre mieux leur stock de bouteilles. Le consommateur final est trompé par cette pratique interdite et paye au prix fort un produit qui ne correspond pas à celui attendu. Le marquage isotopique va permettre d'identifier l'origine de la provenance d'un vin, de son domaine, d'une parcelle et les informations enregistrées concernant les éléments majeurs tels que C, H, O, N, S dans la base de données vont permettre de déterminer une période de cycle de production. Les variations isotopiques des éléments majeurs dans le temps seront suffisamment discriminantes pour une datation précise de chaque cycle.

Concernant le degré de variabilité acceptable, chaque code unique nouvellement créé est défini dans un intervalle de valeurs, ou bien le calculateur contient des instructions pour appliquer un certain niveau de variabilité, pour chaque élément isotopé, qui tient compte notamment de l'adjonction de marqueurs en fonction du type de liquide ou du sous-produit. D'où la présence dans le calculateur de données liées aux liquides et sous-produits dérivés. De manière générale, les marqueurs se retrouve sur l'ensemble du processus et quel que soit le type de liquides. Les quantités d'isotopes ajoutés dans le cadre du processus lié à la transformation d'un liquide naturel pourra être de 1-5-10/1000 par rapport aux valeurs finalement mesurées. Des données plus précises peuvent être générées et enregistrées dans le calculateur, ces données pouvant être issues d'expérimentation, notamment d'expérimentation sur le lieu de récolte ou de transformation ou d'un sous ensemble. Le terme « sensiblement » utilisé ici tient compte de cette variabilité, simplement la méthode de l'invention autorise une variabilité qui ne remet en cause ni la génération d'un code unique (avec les variations isotope par isotope), ni la capacité de comparer efficacement une signature mesurée dans un échantillon et un code unique. « Sensiblement » peut notamment signifier un écart d'au plus 5, 4, 3, 2, 1, 0,5 ou 0,1% pour la concentration ou le ratio.

L'invention permet d'identifier l'origine des produits issus de liquides naturels ou transformés

Comme il sera vu plus loin, la méthode peut permettre, et le calculateur peut aussi être programmé, pour permettre d'identifier le cycle du lieu de récolte ou de transformation (date de production d'un lot ou d'un millésime). Dans ce cas la mesure des majeurs et éventuellement de leurs isotopes (C, H, O, N, S) dans les liquides ou sous-produits à chaque cycle, qui varient de cycle en cycle du fait des variations dans les liquides, sont enregistrées cycle après cycle dans le support d'enregistrement du calculateur, et le calculateur est programmé pour pouvoir comparer les valeurs de ces majeurs dans un échantillon d'un liquide ou d'un sous-produit, et de faire le lien avec des valeurs de majeurs entre différents cycles enregistrés dans le support d'enregistrement.

**Méthode permettant d'imposer un code unique**

La méthode permettant d'imposer un code unique propre aux liquides d'un lieu d'origine. Ce code unique est la combinaison de ratios d'isotopes stables naturels et de ratios d'isotopes stables imposés. Cette méthode est notamment adaptée à la mise en oeuvre de la méthode d'identification isotopique précitée. Elle peut comprendre en particulier:

i) l'analyse de l'abondance d'éléments chimiques et d'isotopes stables d'un ou plusieurs éléments chimiques a) dans un liquide d'au moins un cycle productif d'un lieu d'origine, ou b) dans un sous-produit de ce liquide, l'on obtient ainsi la signature géochimique basale (SGB) du liquide ou du sous-produit,

2i) la sélection d'un ou plusieurs, de préférence plusieurs, notamment au moins 3, éléments chimiques ayant des isotopes stables, de préférence choisis parmi les éléments chimiques présents dans la SGB ou dans la liste donnée ci-après,

3i) l'ajout au liquide de ce cycle productif ou de cycles productifs ultérieurs du lieu d'origine, d'un ou plusieurs isotopes stables des éléments chimiques sélectionnés. Cet ajout peut prendre la forme d'une solution aqueuse, d'une formulation solide ou de toute autre forme d'adjonction isotopée, comprenant une quantité déterminée d'un ou plusieurs isotopes stables du ou des éléments sélectionnés, par exemple un mélange de ces isotopes selon une abondance déterminée (e.g. ratios des isotopes d'un même élément) des isotopes stables des éléments sélectionnés. Cet ajout ou cette abondance est calculée (notamment par le calculateur) afin de conférer un code isotopique propre aux liquides ou à leurs sous-produits du lieu d'origine.

Ce code isotopique unique comprend des ratios d'isotopes issus de la SGB (non concernés par l'adjonction d'isotopes, donc que l'on ne fait pas varier), et des ratios imposés d'isotopes d'éléments (éléments de préférence présents dans la SGB) résultant de l'adjonction imposée d'isotopes.

Etape i)

Lorsque le liquide d'origine est le produit fini lui-même, on analyse de préférence l'abondance des éléments et/ou des isotopes stables conformément à la caractéristique a).

Lorsque le liquide d'origine est transformé par des opérations qui conservent substantiellement la matière sèche du liquide d'origine, par exemple concentration, dilution, vinification, etc., les concentrations et/ou les ratios sont sensiblement constants entre le liquide d'origine et le sous-produit fini, et l'on peut opérer selon les caractéristiques a) ou b).

Dans une modalité cependant, le liquide ou son sous-produit peut récupérer des éléments et/ou des isotopes au cours de son cycle productif, par exemple le vin ou les spiritueux peuvent acquérir des éléments et/ou des isotopes du récipient qui les contiennent, par exemple du tonneau en matière naturelle (bois, etc.). Dans ce cas on peut travailler sous la caractéristique a) ou b), mais en se plaçant à un moment donné du cycle productif, de préférence à la fin de ce cycle productif avant une possible commercialisation ou stockage dans un récipient neutre du point de vue échanges d'éléments et/ou d'isotopes, par exemple au moment de la mise en bouteille.

En variante, qui a une importance dans le domaine du vin et des spiritueux, on peut ne pas se contraindre à une telle mesure en fin de cycle productif. On peut le faire avant et notamment le plus tôt possible dès que l'on atteint un taux d'isotopes stables issu de l'ajout d'isotopes. Ensuite, l'on tient compte des variations qui vont se produire jusqu'à la commercialisation. Ainsi on connaît d'avance ces variations et le modèle en tient compte. Il en tient compte en intégrant dans le code unique l'impact de ces variations. Par exemple, si le tonneau apporte des éléments et/ou des isotopes stables de tel ou tel élément selon une quantité connue ou analysable, le code intègre cet élément, en le combinant à la signature isotopée atteinte au moment de l'ajout des isotopes. Ceci permet notamment d'empêcher (ou de permettre de tracer) toute falsification du liquide au cours de la transformation en aval du marquage.

Lorsque le liquide d'origine est transformé par un processus entraînant l'élimination de fractions du liquide d'origine et de certains éléments ou isotopes stables, modifiant les concentrations ou les ratios du liquide d'origine, dans ce cas le modèle de l'invention en tient compte. On préfère alors employer la caractéristique b) et donc calculer la SGB du sous-produit lui-même. En variante, on utilise la caractéristique a), mais en connaissant l'impact moyen de la transformation sur la SGB et le modèle en tient compte.

La concentration en éléments et/ou en isotopes dans les liquides ou sous-produits dérivés est naturellement liée au lieu de culture ou de récolte, par les apports du sol et de l'eau de ruissellement disponible. Dans un mode de réalisation, pour la définition du SGB, en dehors de toute adjonction d'isotope, dans un cycle productif différent ou avant ajout d'isotopes, les liquides peuvent être analysés en concentration ou ratios des éléments et/ou isotopes à chaque cycle de culture et de récolte, ou de transformation. De préférence, pour la définition du SGB, on effectue en outre des analyses de contrôle à différentes étapes du processus de transformation sur les liquides ou sous-produits dérivés, afin de vérifier la présence des isotopes que l'on a choisi de faire varier lors d'un autre cycle productif ou à une étape ultérieure, ainsi que leur concentration ou leurs ratios.

La SGB peut correspondre sensiblement à la signature isotopique qu'aurait un liquide ou un sous-produit dérivé d'un lieu d'origine, produit ou traité de la manière traditionnelle de ce lieu d'origine, sans intervention d'une adjonction isotopée.

Les végétaux et animaux sont susceptibles d'être alimentés en minéraux et eau, ayant une signature isotopique, et/ou une signature élémentaire, notamment en majeurs, qui peut varier au cours des cycles de production.

Dans ce cas, on peut vouloir disposer d'un code unique, ou une fraction de code unique, valable sur plus d'1 cycle de production. Dans ce cas, on peut ajouter une ou des analyses régulières (par exemple 1 par cycle, en concentration) du sol et/ou de l'eau de ruissellement. Le modèle peut ensuite tenir compte des variations constatées, il peut les juger insignifiantes pour l'obtention du code unique ou de sa fraction, ou il peut décider d'apporter une correction à l'alimentation isotopée. On peut aussi tenir compte des apports potentiels des récipients contenant les liquides et sous-produits, comme les tonneaux en bois dans le cas du vin et des spiritueux.

De même, dans une gestion long ou moyen terme des produits originaires d'un lieu d'origine, ou pour marquer un millésime par exemple, on tient compte des variations apportées par le sol et/ou l'eau de ruissellement, ou par un récipient (e.g. tonneau de bois), mesurées comme précédemment, pour modifier le code unique en ce qui concerne les éléments variables, en général les majeurs pour tenir compte de ces apports variables et de leur impact dans le liquide d'origine ou le produit transformé compte tenu du taux d'accumulation concerné.

Etape 2i)

Dans un mode de réalisation, on peut enrichir en un ou plusieurs isotopes, ou appauvrir en un ou plusieurs isotopes, ou combiner enrichissement ou appauvrissement, par rapport à ce qu'un régime non modifié apporterait aux liquides ou sous-produits transformés lors d'un cycle donné. L'appauvrissement en un isotope d'un élément s'obtient en effectuant un ajout d'un ou plusieurs autres isotopes de ce même élément, ce qui se traduit par un ratio appauvri en l'isotope qui n'a pas fait l'objet d'une adjonction.

On verra plus loin que l'on peut mesurer les ratios d'isotopes d'un nombre plus ou moins important d'éléments, notamment parmi les éléments permettant une plus fine discrimination géographique. Plus ce nombre est grand, plus la signature de base SGB représente déjà un degré de précision important concernant un lieu d'origine, plus il est possible de réduire le nombre d'éléments pour lesquels on va imposer une modification du ratio d'isotopes. C'est pourquoi on dira que l'on commence de préférence à une variation d'au moins 1, 2 ou 3 éléments, mais l'homme du métier comprend maintenant parfaitement que l'on peut faire varier ce nombre, et en particulier augmenter ce nombre pour augmenter la précision si besoin est.

De préférence, à l'étape 2i, on définit un temps auquel les liquides ou les produits transformés reçoivent l'adjonction isotopée, de manière à obtenir, au moment de la mise en bouteille ou en emballage, des liquides ou des sous-produits dérivés ayant acquis le code unique propre au lieu d'origine. Cet instant est adapté pour que le code unique soit présent jusqu'au moment de la mise en bouteille ou en emballage. Si l'on veut donner des précisions, ce temps est de préférence choisi en fonction du bénéfice attendu tels que l'adjonction de marqueurs le plus en amont du processus de culture ou de récolte pour le vin, lors de la réduction en alcool pour les spiritueux, lors de l'intégration de la liqueur de champagne ou dès la mise en cuve pour le lait.

Choix des éléments

De préférence, on associe certains éléments et leur répartition isotopique à des fonctions de localisation et/ou d'identification de cycle ou pour certains liquides à des millésimes. Ainsi, certains éléments et leurs variations isotopiques sont reliés à une localisation géographique grossière (e.g. régionale), d'autres à une localisation fine (e.g. lieu de culture ou de récolte ou un sous-ensemble, parcelles, etc.), d'autres au cycle de culture ou de récolte. Enfin, certains éléments et leurs ratios isotopiques sont des marqueurs dont on impose la variation pour la finalisation du code unique.

L'isotopie naturelle permet d'effectuer une géolocalisation régionale. Elle est généralement basée sur des analyses de ce que l'on appelle les 5 majeurs (C, H, O, N, S) et l'interprétation des concentrations et/ou des ratios isotopiques effectués à partir d'un instrument analytique adapté, de préférence de type IRMS. Les concentrations de ces majeurs (ou les ratios des isotopes de de ces majeurs) sont de préférence toutes mesurées, ou alors au moins 2, 3 ou 4, selon le degré de finesse apporté pour un type de liquide d'origine. De préférence, on choisit de ne pas faire varier leurs concentrations ou ratios afin de conserver leur capacité à donner ou marquer une localisation géographique régionale. Ces éléments et leurs isotopes stables sont alors de préférence partie de la SGB.

Les majeurs (C, H, O, N et S) peuvent aussi être des marqueurs de production, en ce sens que leurs concentrations et/ou leurs ratios isotopiques varient avec le sol, les végétaux, et l'eau de ruissellement, en fonction du moment de l'année et des variations de composition du sol, des végétaux et de l'eau de ruissellement dans l'année. Dans un mode de réalisation, les concentrations et/ou les ratios dans les végétaux et l'eau sont mesurés ou connus pour chaque cycle de culture, de récolte, ainsi que leur impact sur la signature isotopique du liquide ou sous-produit dérivé à la mise en bouteille ou en emballage. Cet élément de la signature apporté par C, H, O, N et S (ou un sous-ensemble discriminant de 2, 3 ou 4) permet alors, sur analyse du liquide ou de sous-produits, de remonter (en plus de l'origine géographique, du lieu de culture, de récolte ou de transformation), jusqu'au cycle de culture, de récolte ou de transformation, et plus précisément la date du lot, du millésime, en fonction de la nature du liquide. Le Millésime est une forme de datation. Par exemple, l'analyse de 2, 3, 4, de préférence des 5 majeurs va permettre d'identifier de manière unique le millésime. Notamment, l'analyse de 2, 3, 4, de préférence des 5 majeurs combiné aux autres éléments du code unique (ou au code unique n'intégrant pas les majeurs) va permettre d'identifier de manière unique le millésime d'un domaine donné. En matière de vins, on peut donc considérer qu'il y a d'un côté le code unique permettant d'identifier un domaine parmi d'autres, et les majeurs pour rapporter le millésime au niveau du domaine. De manière préférée, c'est le recoupement des deux éléments de signature qui contribue à l'identification sûre du domaine et du millésime.

Afin de fournir une origine géographique encore plus précise et de concourir à l'obtention d'une signature incluant une origine géographique plus fine, dans un mode de réalisation préféré, on a recours à des mesures de concentrations d'éléments et/ou d'isotopes d'éléments capables de signer une origine géographique plus fine que les majeurs. Ces éléments sont ceux des deux autres listes évoquées supra. Parmi ces listes, il y a certains éléments tels que le Sr, B, Li, Ca, Na, Mg, K, F, P, Cl, As, Pb, Cd qui permettent de discriminer des lieux géographiques proches, voire contigus. De préférence, on mesure ces éléments (tous ou un nombre représentatif et discriminant, notamment au moins 5, 6, 7, 8, 9, 10, 11, 12 ou tous), en concentration et de préférence en isotopie. Sr, B et Li sont des éléments traces géographiques, marqueurs du sol et de l'eau, et se retrouvant le végétal (la vigne, fruits, les herbes, tout ce qui touche à la botanique) dans des concentrations et ratios spécifiques. De préférence on mesure aussi les ratios de ces 3 éléments. Ca, Na, Mg, K, F, P et Cl sont des éléments trace majeurs apportés par le sol, les végétaux et l'eau. De préférence, on choisit de ne pas faire varier leurs concentrations ou ratios afin de conserver leur capacité à donner ou marquer une localisation géographique fine Il en va de même avec les éléments Sr, B et Li ou les éléments As, Pb et Cd. Ces éléments et éventuellement leurs isotopes stables sont alors de préférence partie de la SGB.

Mais pour réellement distinguer des liquides d'origine ou transformés issus d'un lieu d'origine ou d'un sous-ensemble d'un lieu d'origine (par exemple aussi lieu de cultures ou de récoltes dans une même localité, parcelles contiguës), on construit le moyen de signature unique (dite imposée) via des éléments traces (isotopes stables) dont on va faire varier/doper l'abondance naturelle. Pour ce faire on fait varier les ratios de certains isotopes, de préférence d'autres éléments que les majeurs. On choisit de faire varier les concentrations, donc les ratios d'isotopes pour un nombre d'éléments suffisant pour la discrimination, et on peut notamment se fixer un minimum ou un total de 1, 2, 3, 4, 5, 6, 7, 8, 9 ou 10 éléments. Dans la mesure où l'on peut ne faire varier les isotopes que pour un nombre restreint mais suffisant d'éléments, les autres éléments des groupes (2), (3) et (4) peuvent être conservés et mesurés pour être intégrés au code unique. Avantageusement, on choisit de faire varier les ratios d'isotopes dont les éléments sont présents naturellement dans les liquides issus d'un lieu d'origine (donc dans la SGB).

De préférence, la méthode comprend, au cours d'un cycle de culture, de récolte ou de transformation, au moins une analyse de l'adjonction isotopée, pour détecter une éventuelle variation ou dérive de l'abondance des isotopes stables des éléments sélectionnés.

De préférence, la méthode comprend l'utilisation d'un calculateur électronique dans lequel sont enregistrés les codes uniques propres à d'autres lieux d'origine, préalablement déterminés et enregistrés. Le calculateur électronique programmable est de préférence pourvu d'une unité logique programmable, d'un support d'enregistrement d'informations et d'une interface d'échange de données raccordés entre eux par un bus de données interne. Le calculateur électronique peut également comporter une interface homme-machine.

De préférence, les données mesurées pour établir la SGB d'un lieu d'origine sont entrées dans le Calculateur, ce dernier, par son unité logique programmable, étant à même de déterminer le SGB ou passeport géochimique, qui établit la base de départ de référence pour un site donné. Ces données comprennent notamment :

- le résultat des mesures de concentration d'éléments chimiques ;
- le résultat des mesures de concentration d'isotopes stables d'un ou plusieurs éléments ;
- le résultat des mesures de ratios des isotopes stables de certains éléments constitutifs de la SGB ;
- le choix d'application de la caractéristique a) ou b) ;
- des données relatives à la disponibilité de divers isotopes stables de divers éléments potentiellement présents dans la SGB, choisis dans la liste des isotopes que l'on peut faire varier;
- les coûts de ces isotopes ; et/ou
- une discrimination entre les 3 groupes d'éléments évoqués plus haut, à savoir une classification des ratios pour chacun des groupes séparément.

Dans un mode de réalisation, le calculateur stocke les données du TA des lieux d'origine, et a de préférence des moyens de calcul permettant d'établir une corrélation entre une variation d'abondance d'éléments d'isotopes et l'adjonction isotopée, pour l'obtention du code unique au moment de la mise en bouteille ou en emballage, respectivement de la culture, récolte ou production. Le TA est dépendant de la quantité mesurée dans les végétaux, l'eau de ruissellement ajoutée au cours de leur cycle naturel de production, et ceci, le calculateur peut en tenir compte pour calculer l'adjonction isotopée à réaliser.

De préférence, le calculateur stocke le SGB des liquides ou sous-produits dérivés. Il stocke aussi les codes uniques propres à d'autres lieux d'origine (de culture, récolte, transformation ou d'autres sous ensemble), qui ont été établis à une période antérieure. Le calculateur, grâce à sa programmation et ses données enregistrées et/ou entrées par l'utilisateur, peut calculer et proposer à l'utilisateur une variation d'abondance des isotopes pour définir l'adjonction isotopée et la méthode d'intégration de cette adjonction isotopée (i.e. temps par rapport à la date de mise en bouteille ou en emballage, voire de vendange) pour conférer aux liquides issus d'un lieu d'origine, le code unique propre au produit fini (par exemple avant passage en tonneau ou au moment de la mise en bouteille ou en emballage).

Suivant une caractéristique avantageuse, le calculateur intègre le code unique attribué à chaque lieu d'origine, il contient notamment tous les codes uniques des lieux d'origine sur lesquels le modèle a été déployé. Chaque code est stocké dans la base de données et peut être modifié pour mise à jour ou ajout de compléments. Ce code est notamment constitué de la SGB et des variations d'isotopes imposées. Il peut être intégré à ce qu'on appelle le passeport géochimique, qui peut comprendre en plus des éléments additionnels, la désignation de l'appellation, d'une parcelle, d'un domaine, de cépage ou toutes autres informations spécifiques liées aux liquides, des variations isotopiques au cours des différents cycles de culture, récolte, transformation variations par exemple liées à la saison, avec notamment le variations isotopiques dans l'eau de ruissellement, à la qualification bio (« organic ») ou non, aux pratiques de culture, de récolte, de transformation, de manière générale l'ensemble des éléments référant à un site donné. Toutes ces données peuvent être consultées.

Les variations d'abondances des isotopes requis, pour l'obtention du code unique, peuvent avantageusement être minimes et un écart entre 1-/1000 à 3/1000 pourra être effectué.

De préférence, le calculateur connaît (l'utilisateur ayant enregistré ces données) et tient compte d'un ou plusieurs, de préférence la totalité des variables suivantes :

- L'origine végétale d'où sont issus les liquides ou sous-produits dérivés,
- la durée du processus pour obtenir un liquide naturel ou transformé avant la mise en bouteille ou en emballage,
- le temps d'une adjonction isotopée,
- les apports d'isotopes stables par un récipient ou une étape de processus de transformation.

Il peut aussi connaître :

- le taux d'accumulation TA des liquides ou sous-produits dérivés dans les conditions de culture, récolte ou transformation,
- l'apport éventuel d'éléments non contrôlés (par exemple en culture, récolte en plein air, pluie pour les végétaux),
- données relatives au sol (notamment dans les cultures bio (« organic »).

Une fois la phase de caractérisation du site réalisée et enregistrée dans le calculateur, on peut avantageusement, avant de définir l'adjonction isotopée et son temps d'adjonction, effectuer une analyse de contrôle sur les liquides d'origine, afin de vérifier que ce que la SGB contient comme isotopes stables et leurs concentrations/ratios, est toujours valide.

Dans un mode de réalisation, le code unique intègre une signature isotopique de plusieurs éléments rares, notamment au moins 5, 8, 12, ou la totalité des éléments du groupe (4). On peut préciser que ces éléments rares sont principalement associés à la localisation géographique de lieux de culture, de récolte ou de transformation, notamment au niveau continent, pays ou région.

Dans un mode de réalisation, le code unique intègre une signature élémentaire et/ou une signature isotopique d'un ou plusieurs éléments, notamment au moins 5, 10, 15, 20, 25, 30, ou la totalité des éléments du groupe (2) et/ou au moins 3, 6, 8, 10 ou la totalité des éléments du groupe (3). Cet élément de signature est notamment est rapporté à l'identité d'un lieu de culture, de récolte, de transformation ou de son sous-ensemble.

De préférence, le code unique intègre une signature élémentaire et/ou isotopique des éléments C, O, N, H, S, qui est notamment rapportée via la végétation à l'origine des liquides et des sous-produits dérivés. De préférence on ne les fait pas varier.

Dans un mode de réalisation préféré, le code unique comprend des concentrations d'éléments et/ou d'isotopes stables d'éléments majeurs, à savoir C, O, H, N et/ou S, obtenus après mesure des concentrations (C3) ou de ratios (R3) de ces isotopes dans un liquide ou un sous-produit dérivé. Les éléments (C, H, O, N et S) sont les éléments dits majeurs. Leur taux peut notamment varier d'un cycle à un autre, notamment au cours d'une même année, et cette variation peut être connue (on mesure les concentrations dans les lots, les cuves, les tonneaux, les millésimes ou autres) et prise en compte dans le code unique, permettant une traçabilité à des niveaux de granularité différents, par exemple d'un lieu de culture de récolte, ou beaucoup plus fins, tels que appellations, cépages, type de production, et même lot (cycle de production).

Le calculateur peut aussi comprendre les données relatives aux concentrations (C3) ou aux ratios (R3) des éléments majeurs ou des isotopes majeurs (C, H, O, N et S) dans les liquides, et leur traduction en concentrations ou ratios que l'on retrouvera chez les produits liquides finis ou les sous-produits dérivés au moment de la mise en bouteille ou en emballage selon l'adjonction ou le taux d'accumulation. Le calculateur est en mesure de calculer les valeurs corrigées prévisibles par correction par le taux d'accumulation chez l'animal ou végétal considéré.

Le calculateur électronique peut également être paramétré avec des données environnementales, des cépages, des mesures du sol, des données de cycle de marquage en fonction de la nature du liquide (vin, champagne, pétillant, spiritueux, lait et produits dérivés), géographiques ou éléments nutritionnels de référence. L'ensemble de ces données permet de générer un code unique adapté au lieu ciblé. Il est également à préciser que le calculateur peut être avantageusement paramétré avec les valeurs nutritionnelles de référence connues, les valeurs de toxicité, et toutes autres critères, qui lui permettront de définir des codes uniques (et donc des recettes et régimes isotopiques) en évitant la génération de codes invalides car s'écartant de ces critères.

Le calculateur calcule et propose les variations d'abondance d'isotopes stables et/ou le temps d'adjonction isotopée pour conférer le code unique au moment de la mise en bouteille ou en emballage. De préférence, le calculateur est programmé pour déterminer ces variations d'abondance de manière optimisée en termes de prix et/ou de disponibilité des isotopes. L'utilisateur peut ainsi enregistrer des données relatives à ces variables variation d'abondance, prix et disponibilité, et les tenir à jour au cours du temps, afin que le calculateur gère au mieux la définition des éléments et des isotopes à faire varier, donc à produire l'adjonction isotopée d'un liquide. Le critère que respecte toujours le calculateur est de définir les éléments et leurs isotopes, et leurs concentrations ou ratios, pour définir un nouveau code unique, différents de ceux déjà établis pour d'autres lieux d'origine.

De préférence, pour ajuster les ratios d'isotopes de l'adjonction isotopée, on fait varier (ou le calculateur propose de faire varier) l'abondance d'isotopes stables des éléments chimiques selon les règles exposées plus haut

On peut décrire les modes de réalisation suivants, avec une stratégie d'utilisation des éléments en fonction des domaines, appellations et de la taille des parcelles afin d'être en capacité de garantir une traçabilité sur le long terme.

1. Les productions de liquides ou sous-produits dérivés en très grande quantité (Dizaines de millions d'hectolitres de lait ou de sous-produits dérivés notamment issus du lait). On sélectionne des éléments traces (parmi Zn, Se, Mo, Si, Ti, Sn, Fe, Cr) produits par centrifugation. On peut enrichir en isotopes ou appauvrir, cette dernière solution étant plus favorable d'un point de vue économique. Le marquage s'effectuera de préférence en amont du processus de transformation.

2. Les productions de liquides pour des volumétries importantes (Milliers à millions de litres de vins, spiritueux, pétillants, champagnes). On sélectionne des éléments traces (parmi Zn, Mo, Si, Ti, Sn, Cr) produits par centrifugation. On peut enrichir en isotopes ou appauvrir, cette dernière solution étant plus favorable d'un point de vue économique. Le marquage se fera en fonction de la nature du liquide (par exemple, pour les spiritueux, au moment du rajout de l'eau pour abaisser le taux d'alcool, pour les vins juste au moment où les grains macèrent après les vendanges, pour les champagnes dans la liqueur de champagne, pour les pétillants, juste avant la mise en bouteille).

Les facteurs clés également retenus sont la solubilité et la répartition des éléments en fonction des liquides. Les mises en oeuvre sont très importantes car elles garantissent l'efficacité du marquage qui se réalise via l'encodage d'une solution aqueuse. Ceci permet d'optimiser l'usage et les quantités d'isotopes stables requis.

On peut aussi décrire les modes de réalisation suivants pour les liquides en fonction du type et du lieu de culture, de récolte ou de transformation (vigne, viticulture et vinification, fourrage, agriculture et traitement du lait, maraichère, fruit, traitement des fruits...)

En fonction du niveau d'enrichissement de l'isotope choisi, cela fait varier l'abondance naturelle des autres isotopes d'un même élément. L'analyse des ratios isotopiques d'un même élément permettra de mettre en évidence le ratio à utiliser déterminant la présence du ou des marqueurs et sa/leur valeur en concentration.

Avec la connaissance des divers éléments et ratios isotopiques, il est possible de se contenter de faire varier (et donc d'apporter les ratios d'isotopes correspondants, enrichis et/ou appauvris) les ratios de seulement 1, 2, 3, 4, 5, 6, 7, 8, 9, 10 éléments.

Les isotopes peuvent être apportés de toute manière connue. On peut utiliser les chlorures, les sulfates et les oxydes. Chlorures et sulfates sont généralement solubles dans l'eau. Ils peuvent être ajoutés simplement à l'eau de boisson ou à tout autre liquide. Les oxydes sont généralement solides, mais peuvent aussi être ajoutés à l'eau d'arrosage ou à tout autre liquide.

De préférence, les quantités d'isotopes apportées aux liquides ou sous-produits sont dans les limites autorisées par les Valeurs Nutritionnelles de Référence (VNR) et dans les limites des valeurs de toxicité. Le calculateur peut donc être programmé avec ces informations sur les VNR et les limites à ne pas dépasser en termes de toxicité, il intégrera ces données dans ses préconisations et calculs.

De préférence, les quantités d'isotopes apportées aux liquides ou sous-produits dérivés sont ajoutés en tenant compte du taux d'accumulation (TA) de ce ou ces isotopes dans le liquide en fonction du temps choisi dans le processus avec l'adjonction isotopée. Comme on l'a vu, le calculateur peut intégrer cette connaissance du TA et en tenir compte comme décrit.

Dans un mode de réalisation, les liquides ou des sous-produits dérivés sont marqués exclusivement ou essentiellement exclusivement avec l'adjonction isotopée.

De préférence, les liquides issus d'un lieu d'origine sont marqués avec l'adjonction isotopée lors d'une étape clé du processus de transformation avant la mise en bouteille ou en emballage. Le calculateur est apte à proposer une méthode d'adjonction isotopée qui convient à l'appellation, aux cépages ou aux variétés de lait considérés, etc. selon des critères propres enregistrés dans son programme.

Le liquide, de composition isotopique connue, peut être utilisé comme tout ou partie d'une adjonction aqueuse isotopée destinée à un liquide d'origine géré par le modèle (M) selon l'invention. Donc, dans un mode de réalisation de la méthode pour imposer un code unique à un liquide d'origine d'un lieu d'origine, on ajoute le volume requis de liquide isotopé ou la quantité requise d'isotopes au liquide d'origine.

L'invention a aussi pour objet un support d'enregistrement d'informations. Le support d'enregistrement peut contenir des instructions exécutables programmées pour mettre en oeuvre un procédé conforme à l'invention lorsque ces instructions sont exécutées par un calculateur électronique.

L'invention a aussi pour objet un calculateur électronique pour la mise en oeuvre, ou utilisable pour la mise en oeuvre de la méthode d'identification isotopique selon l'invention. Ce calculateur peut comporter une unité logique programmable et un support d'enregistrement d'informations contenant des instructions logicielles adaptées pour, lorsqu'elles sont exécutées par l'unité logique, mettre en oeuvre des étapes de comparaison d'un profil de concentrations ou de ratios d'éléments chimiques et d'isotopes stables, d'un échantillon issu d'un liquide (une bouteille ou un emballage contenant un liquide ou sous-produits dérivés), sous forme de concentrations (C2) ou de ratios (R2) , avec des profils enregistrés sous forme de codes uniques chacun propre à un lieu d'origine, et de détermination si le produit liquide ou le sous-produit dérivé a un profil sensiblement égal à un code enregistré et donc indication du lieu d'origine, ou que le produit liquide ou le sous-produit dérivé ne provient d'aucun lieu d'origine enregistré dans le modèle ou encore que le liquide a été falsifié. Le support d'enregistrement d'informations (4) comprend notamment en mémoire les codes uniques d'au moins un liquide de référence (notamment un vin ou spiritueux), ce code unique étant représentatif d'une appellation, d'un domaine et/ou d'un millésime et comprenant, pour chaque liquide de référence (vin ou spiritueux notamment), les concentrations d'au moins 5, 6, 7, 8, 9, 10, 11, 12 ou la totalité des éléments du groupe (3) Rb, Sr, B Li, Ca, Na, Mg, K, F, P, CI, As, Pb, Cd, et d'au moins 5, 10, 15, 20, 25, 30, ou la totalité des éléments du groupe (2) : Be, AI, V, Cr, Mn, Fe, Co, Ni, Cu, Zn, Ga, Ge, Se, Y, Zr, Nb, Mo, Rh, Pd, Ag, Sn, Sb, Te, I, Ba, Hf, Ta, W, Re, Ir, Hg, Ti, Si, et des concentrations ou des ratios d'isotopes d'au moins 1, 2 ou 3 des éléments du groupe (2') AI, Mn, Fe, Co, Cu, Zn, Mo, Sn, Ti, Si. Il comprend aussi de préférence les concentrations (C3) ou les ratios (R3) des éléments majeurs (C, H, O, N et S), éventuellement leurs isotopes stables.

Le code unique enregistré pour les liquides de référence comprend notamment des concentrations d'isotopes stables d'au moins 1, 2 ou 3 des éléments du groupe (2') AI, Mn, Fe, Co, Cu, Zn, Mo, Sn, Ti, Si ; de préférence les concentrations d'au moins 2 isotopes stables d'au moins 1, 2 ou 3 des éléments Fe, Cu, Zn, Mo, Sn, Ti, Si. En particulier, il comprend les concentrations d'au moins 2 isotopes stables d'au moins 1 élément parmi Fe et Zn, notamment 56Fe et 57Fe, et/ou 66Zn et 68Zn.

Le code unique comprend notamment les concentrations des éléments Rb, Sr et B.

Le code unique comprend notamment les concentrations des éléments Li, Ca, Na, Mg, K, F, P, CI, As, Pb, Cd.

D'autres caractéristiques du calculateur électronique ont été décrites supra.

Le calculateur peut encore comprendre toutes les fonctionnalités, dispositifs et programmations nécessaires à l'accomplissement de taches décrites ici et qui lui sont dévolues :

- Le calculateur est la garante de l'intégrité des codes uniques.
- Le calculateur électronique reçoit en entrée des résultats des analyses en concentration (Passeport géochimique).
- Tous les éléments traces géographiques, ultra traces, macro traces, et éléments traces (micros) peuvent y être répertoriés.
- Le calculateur contient également la logique et les stratégies applicables au type d'élevage ou de culture.
- Lors de la détermination des variations isotopiques à effectuer par rapport à l'abondance naturelle sur 1, 2, 3 ou plus éléments, le calculateur va s'assurer que le code n'est pas déjà alloué.
- Si le code est déjà alloué et en fonction de la logique et les stratégies applicables au type d'élevage ou de culture, le calculateur va effectuer une variation supplémentaire en fonction d'un indice de dopage (+1/1000 par exemple) jusqu'à ce qu'elle trouve une disponibilité de code.
- S'il s'avérait qu'il y ait un problème de disponibilité, elle va d'abord vérifier les éléments différentiateurs du passeport géochimique et tenir compte d'une valeur ou de plusieurs valeurs et les ajouter à la recette du code unique. Cela permet de ne pas ajouter en général plus de trois marqueurs. On préfère en effet avoir au minimum une signature basée sur la variation de 3 isotopes.

- Une fois la recette/code unique généré, le code est enregistré dans le calculateur.

L'invention va être maintenant décrite plus en détail à l'aide de modes de réalisation pris à titre d'exemples non limitatifs et se référant au dessin annexé.

La figure 1 est un schéma d'un ensemble avec calculateur électronique utilisable pour la mise en oeuvre de l'invention.
La figure 2 est un ordinogramme d'un procédé permettant d'imposer un code unique à des liquides.
La Figure 3 est un extrait de graphe montrant, pour différents vins et spiritueux, les concentrations en éléments chimiques B, Na, Mg, Al, Ca, Mn, Cu, Rb et Sr, et en isotopes 56Fe, 57Fe, 66Zn et 68Zn, obtenus par spectrométrie de masse. L'intérêt du graphe ne se situe pas dans les valeurs précises mesurées, mais dans le fait de montrer le fait que les valeurs varient d'un produit à un autre.
La Figure 4 est un graphe montrant les variations du ratio 68Zn/67Zn dans le vin en fonction du dopage réalisé en 68Zn. Abscisse : DDG est le nom du vin, puis indication du % de dopage en 68Zn. Ordonnée : valeur au spectromètre de masse.
Les figures 5 à 7 sont des graphes MS montrant l'évolution des ratios des isotopes 54Fe/56Fe, 57Fe/54Fe et sur 57Fe/56Fe avec ou sans correction de l'interférence entre 54Cr et 54Fe, sur des échantillons du vin DDG, et en fonction du dopage isotopique effectué (variation de la concentration d'un isotope par rapport à son abondance naturelle de référence).
Les figures 8 à 10 sont des graphes MS montrant l'évolution des ratios des isotopes 54Fe/56Fe, 57Fe/54Fe et sur 57Fe/56Fe, sur des échantillons du vin DDG, et en fonction du dopage isotopique effectué. Zn-Fe et de DZn-Fe sont des combinaisons de traceurs (code uniques) permettant de démontrer qu'il est possible de marquer/encoder le même liquide avec une combinaison différente dans le même intervalle de valeurs.

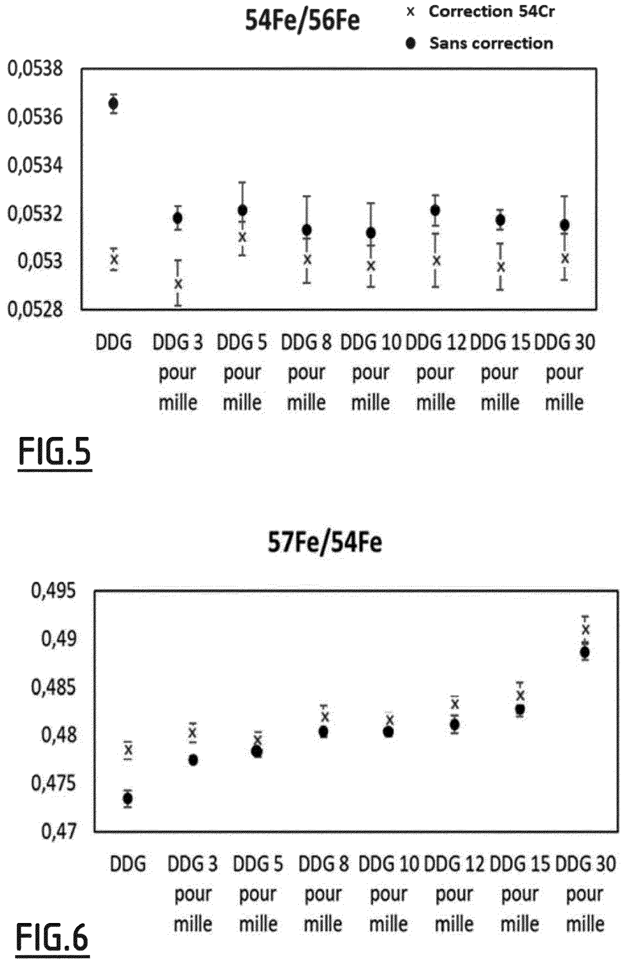

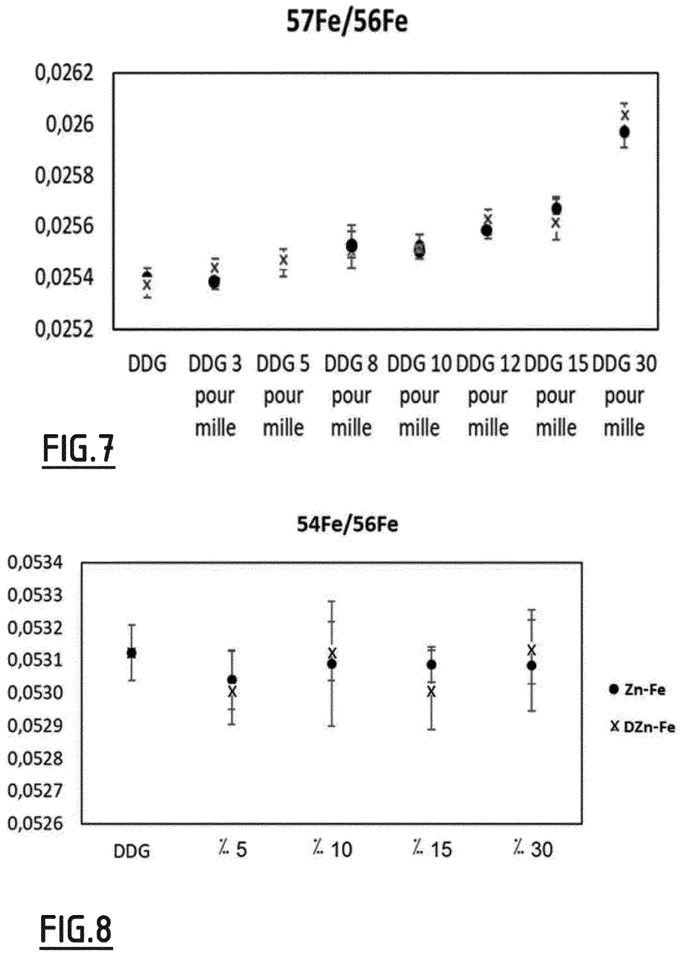

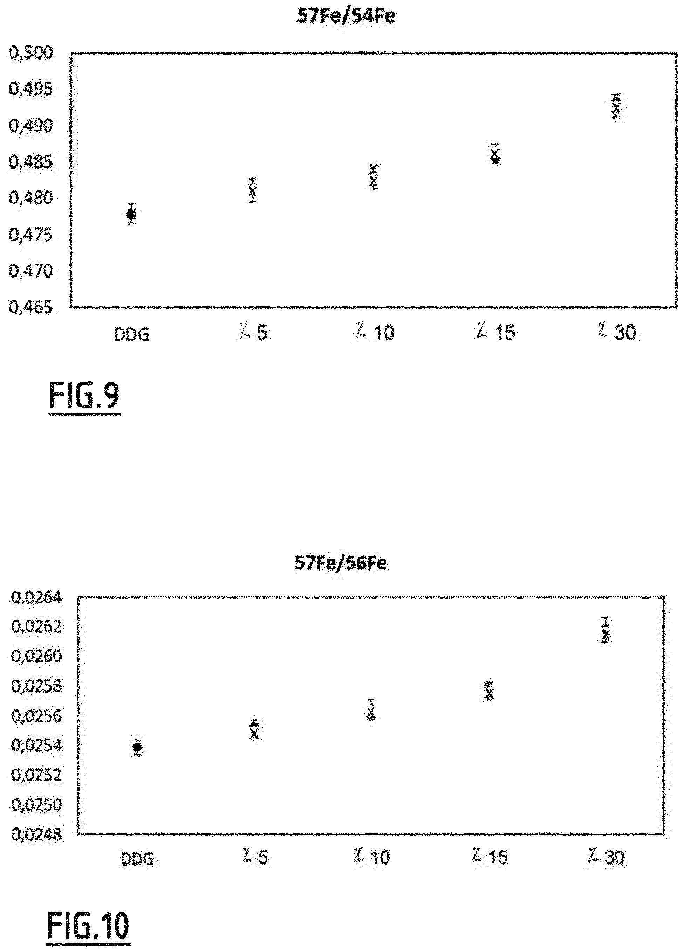

Sur ces figures 5 à 10, les croix se superposent parfois totalement ou partiellement sur les ronds noirs, ce qui explique qu'ils sont peu ou pas visibles, mais ils sont bien présents, en dessous de la croix qui le recouvre.

Exemples :

Exemple 1 : Description d'un ensemble informatique de gestion et de définition des codes isotopiques

L'ensemble 1 comporte un calculateur électronique 2 programmable, pourvu d'une unité logique programmable 3, d'un support d'enregistrement d'informations 4 et d'une interface d'échange de données 5 raccordés entre eux par un bus de données interne. Le calculateur électronique 2 comporte également ici une interface homme-machine 6.

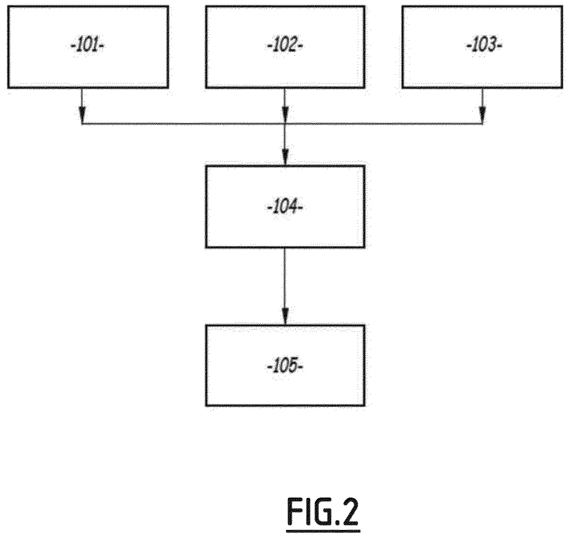

L'unité 3 comporte par exemple un microprocesseur ou un microcontrôleur programmable. Le support 4 comporte ici un module mémoire, par exemple de technologie FLASH ou EEPROM, ou encore un disque dur magnétique. Le support 4 contient des instructions logicielles adaptées pour mettre en oeuvre des étapes du procédé de la figure 2 lorsque ces instructions sont exécutées par l'unité de calcul 3.

L'interface homme-machine 6 comporte ici un écran d'affichage, un outil de saisie de données tel qu'un clavier et un haut-parleur. En variante, l'interface homme-machine 6 peut être réalisée différemment.

Par exemple, le calculateur électronique 2 est un micro-ordinateur ou un dispositif de communication mobile, tel qu'une tablette ou un téléphone. Il peut également s'agir d'un serveur informatique distant, accessible au travers du réseau internet ou d'un réseau informatique dédié. Dans ce cas, l'interface 6 peut être omise et remplacée par une interface de communication dédiée, par exemple un ordinateur, un dispositif de communication tel qu'une tablette ou un téléviseur, qui remplit les mêmes fonctions que cette interface 6 mais qui est physiquement dissociée du calculateur électronique 2.

Le calculateur 2 est notamment programmé pour implémenter un modèle M prédéfini, par exemple grâce à des instructions exécutables stockées dans le support 4.

Le modèle M permet notamment d'imposer aux liquides ou sous-produits dérivés un code isotopique unique propre à un lieu d'origine, et éventuellement à des niveaux de granularité encore plus fins (e.g. appellations, cépages, type de production, éventuellement lot), ce code étant basé sur la nature, les concentrations ou les ratios en isotopes stables d'éléments chimiques. Le modèle (M) permet en outre, le cas échéant, de relier un liquide issu d'un lieu d'origine déterminé, par analyse de concentrations ou de ratios d'isotopes stables, permettant de déterminer un profil de concentrations ou de ratios de ces isotopes stables, notamment par spectrométrie de masse, et de comparer aux codes uniques enregistrés dans le modèle (M).

Les données utilisées par le modèle M peuvent être stockées dans le support 4 et/ou être stockées dans une base de données dédiée accessible par le calculateur 2.

Par exemple, l'interface 5 est adaptée pour acquérir des données d'entrée, par exemple sous la forme de signaux numériques ou analogiques ou sous la forme de structures de données, telles que des valeurs de taux d'accumulation TA et/ou des mesures de concentrations C2 et/ou de ratios R2 d'isotopes stables. Ces données peuvent également être transmises au calculateur 2 par l'intermédiaire de l'interface 6.

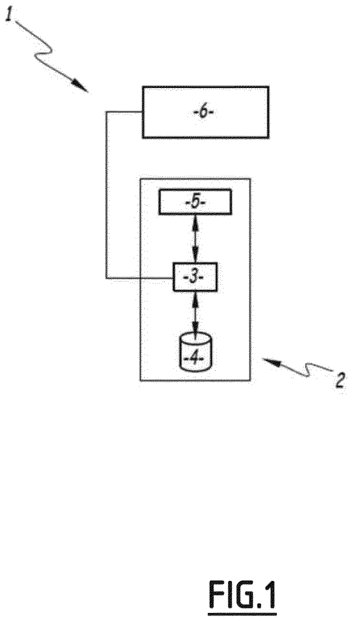

La figure 2, en lien avec la figure 1, décrit de manière schématique un mode de réalisation de la méthode d'imposition de code unique. On procède à la détermination par SM de la SGB du liquide d'origine à l'étape 101, les données sont adressées au calculateur 2, par exemple par l'intermédiaire de l'interface 6.

Le calculateur a en mémoire les codes uniques qui ont été générés pour d'autres lieux d'origine, on identifie cette connaissance lors d'une étape 103 à la figure 2.

En croisant les données obtenues lors des étapes 101, 102 et 103, le calculateur génère lors d'une étape 104 une recette d'adjonction isotopée qui permettra d'obtenir, à la mise en bouteille ou à l'emballage, des liquides ayant le code unique.

Le régime d'adjonction peut être testé et les données conservées dans le calculateur 2, pour une corrélation entre cette opération et l'obtention d'un ratio d'isotopes stables d'un élément au moment de la mise en bouteille ou à l'emballage d'un liquide ou d'un sous-produit dérivé. Des ajustements (en teneur notamment) sont réalisables pour obtenir des ratios d'isotopes exploitables, i.e. avec des différences significatives mesurables par SM à la mise en bouteille ou à l'emballage.

Le calculateur génère la composition de l'adjonction isotopée, l'utilisateur pouvant y accéder par exemple depuis l'interface 6.

En variante, le régime d'alimentation peut avoir été déterminé à l'avance, et le calculateur indique à l'utilisateur la composition de l'adjonction isotopée.

Exemple 2 : Application à un domaine de vins rouge

On se réfère aux figures 1 et 2.

On procède à la détermination de la SGB d'un liquide issu d'un lieu d'origine à l'étape 101, les données sont adressées au calculateur 2, par exemple par l'intermédiaire de l'interface 6. On recueille, puis analyse des échantillons de liquides. Les ratios des isotopes stables des éléments suivants (on a pu déterminer par des analyses préalables leur présence sur le lieu de culture, de récolte de transformation, par exemple par analyse par spectrométrie de masse (SM) sur le liquide lui-même ou sur l'eau de ruissellement, le sol, la végétation (vigne, arbres fruitiers...)) :

- ces 26 éléments: Li, Be, B, F, Na, Mg, Al, Ca, Cr, Mn, Co, Ni, Cu, Zn, Ga, As, Se, Rb, Sr, Mo, Rh, Pd, Ag, Cd, Te, Ba, Ti, Pb, Si,
- ces 15 terres rares : La, Ce, Pr, Nd, Pm, Sm, Eu, Gd, Tb, Dy, Ho, Er, Tm, Yb, Lu.

Les mesures de SM dans l'invention en général et dans cet exemple en particulier, peuvent être effectuées par les méthodes disponibles, notamment :

- la spectrométrie de masse à plasma à couplage inductif, ou ICP-MS (en anglais : Inductively Coupled Plasma Mass Spectrometry),
- la spectrométrie de masse à source plasma et à multi-collection, ou MC-ICPMS (en anglais Multicollector-Inductively Coupled Plasma Mass Spectrometer)
- la spectrométrie de masse à ratio isotopique, ou IRMS (en anglais Isotope-ratio mass spectrometry).
- LA-ICP/MS (Ablation Laser)
- LIBS (Laser Induced Breakdown Spectroscopy (LIBS).

Le TA est connu ou il peut être calculé en étape 102 en marquant les liquides sur un cycle avec des ratios déterminés des isotopes stables des éléments indiqués, puis mise en bouteille ou en emballage, prélèvement de liquides ou de sous-produits dérivés et analyse par SM. Les données sont adressées au calculateur 2, par exemple par l'intermédiaire de l'interface 6.

Le calculateur a en mémoire les codes uniques qui ont été générés pour d'autres lieux d'origine, on identifie cette connaissance lors d'une étape 103 à la figure 2.

En croisant les données obtenues lors des étapes 101, 102 et 103, le calculateur génère lors d'une étape 104 une recette d'adjonction isotopée et un régime de traitement qui permettront, dans ce lieu d'origine d'obtenir, à la mise en bouteille ou en emballage, des liquides ayant le code unique.

Le régime d'adjonction peut être testé et les données conservées dans le calculateur 2, pour une corrélation entre ce régime et l'obtention d'un ratio d'isotopes stables d'un élément au moment de la mise en bouteille ou en emballage. Des ajustements (en teneur notamment) sont réalisables pour obtenir des ratios d'isotopes exploitables, i.e. avec des différences significatives mesurables par SM à la mise en bouteille ou en emballage.

Le calculateur génère la composition de l'adjonction isotopée et/ou le régime de traitement (Processus opérationnel devant être suivi par l'opérateur des lieux), l'utilisateur pouvant y accéder par exemple depuis l'interface 6.

En variante, le régime d'adjonction peut avoir été déterminé à l'avance, et le calculateur indique à l'utilisateur la composition de l'adjonction isotopée.

Pour les liquides, on peut ainsi définir un régime dans lequel l'adjonction isotopée constitue le besoin aqueux de la semaine précédant la mise en bouteille ou l'emballage, étape 105.

Exemple 3 : Application à des vins, dont champagnes, et spiritueux

| Code | Appellation | Year | Color | Alcool |
|------|-------------|------|-------|--------|
| CR | Côtes du Rhône | 2016 | Rouge | 13.5° |
| B1 | Bordeaux | 2015 | Rouge | 12.5° |
| B2 | Côte de Bourg Château Lallibarde (Bordeaux) | 2016 | Rouge | 13.0° |
| M | Muscadet Sèvre et Maine-sur-Lie | 2016 | Blanc | 12.0° |
| AR | Alsace Riesling | 2016 | Blanc | 12.0° |
| BA | Bourgogne Aligoté | 2016 | Blanc | 12.0° |
| DG1-2 | Domaine des Gardes | 2015 | Rouge | 13.0° |
| R1-2 | Tain l'Hermitage | - | Rouge | 12.0° |
| CM | Champagne Mercier | - | Champagne | 12.0° |
| CBN | Champagne Alexandre Bonnet | - | Champagne | 12.5° |
| PSM | Cognac V.S Prince St Mérac | - | Cognac | 40.0° |
| VCC | Viognier, Caprice de Clairmont | 2016 | Blanc | 13.0° |

Par spectrométrie de masse, à l'aide d'un ICP/MS ICAP RQ, pour chaque produit du tableau précédent, on a déterminé les concentrations des éléments chimiques présents, à savoir B, Na, Mg, Al, K, Ca, Mn, Fe, Cu, Zn, Rb, Sr, Li, Be, Sc, Ti, V, Cr, Co, Ni, Ga, Ge, As, Se, Y, Zr, Nb, Mo, Pd, Ag, Cd, In, Sn, Sb, Cs, Ba, La, Ce, Pr, Nd, Sm, Eu, Gd, Tb, Dy, Ho, Er, Tm, Yb, Lu, Hf, W, Hg, Tl, Pb, Bi, Th, U.

C, H, O, N, S pourront être analysés en plus pour la datation de la production.

Sur ces produits viticoles, on s'aperçoit que les terres rares sont quasi inexistantes.

Parmi les éléments Al, Mn, Fe, Co, Cu, Zn, Mo, Sn, Ti, Si, on s'est intéressé en premier lieu à Fe et Zn. La mesure a été conduite dans des conditions permettant de mesurer les concentrations en isotopes $^{56}Fe$, $^{57}Fe$, $^{66}Zn$ et $^{68}Zn$. La présence de ces isotopes stables dans tous les échantillons et leur répartition permet d'envisager de faire varier la concentration de l'un par rapport à l'autre et donc leurs ratios, notamment les ratios $^{56}Fe/^{57}Fe$ et $^{66}Zn/^{68}Zn$, ou encore les ratios de ces isotopes de Zn par rapport à l'isotope majoritaire ($^{64}Zn$) en abondance naturelle.

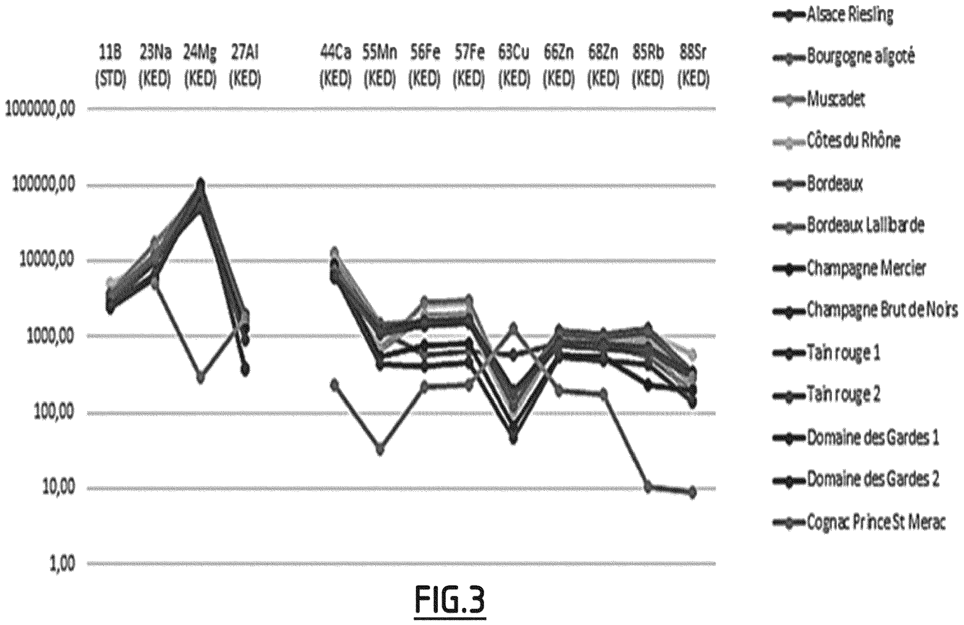

Le graphe de la Figure 3 est un extrait des données obtenues. On voit apparaître les différences de concentrations selon les produits analysés.

Faire varier les concentrations ou les ratios peut être réalisé notamment:

- Par ajout de l'isotope dont on veut augmenter la concentration,
- Par ajout de l'isotope naturel majoritaire,
- Par ajout d'un mélange d'isotopes en abondance naturelle, ou
- Par ajout d'un mélange d'isotopes appauvris en isotope majoritaire.

Une 1ère série a été réalisée afin de définir les meilleures procédures de préparation d'échantillons sur les mesures des rapports d'isotopes analytiques. Les échantillons sont des vins blancs, rouges et de Champagne, et un Cognac.

Ces tests de marquages ont d'abord été effectués sur des échantillons de vins distincts de 10 ml, puis reproduit sur des bouteilles. Le marquage isotopique $^{68}Zn$ a été effectué pour obtenir une variation (dopage) de 30 ‰ et plus, en fonction de la concentration initiale de Zn. Ils étaient uniquement dilués ou minéralisés et dilués avant analyse. Le facteur de dilution est approximativement 20 et la concentration de Zn près de 50 ppb.

| | Tests | | | | | | |
|---|---|---|---|---|---|---|---|
| | 68ZN 0.95 ppm en solution | Volume | Résultats 68/67Zn | Volume | Résultats 68/67 | Résultats 68/66 | |
| BA | Alsace Riesling | 59,8 µl | 39,4 ‰ | 48,2 µl (48,262) | 37,7 ‰ | | |
| M | Bourgogne Aligoté | 56,8 µl | 28,9 ‰ | 60,2 µl (60.168) | 27,0 ‰ | | |
| AR | Muscadet | 47,0 µl | 26,8 ‰ | 60,2 µl (60168) | 26,1 ‰ | | |
| CR | Côtes du Rhône | 50,0 µl | 31,4 ‰ | 60,2 µl (60,168) | 30,8 ‰ | | |
| B1 | Bordeaux 1 | 59,4 µl | 29,7 ‰ | 60,2 µl (60,168) | 28,1 ‰ | | |
| B2 | Bordeaux 2 | 57,0 µl | 28,4 ‰ | 55,8 µl (55,786) | 28,7 ‰ | | |
| CM | Champagne Mercier | | | 33,4 µl (33,419) | 29,4 ‰ | 26,9 | |
| CBN | Champagne A. Bonnet | | | 31,0 µl (31,082) | 29,4 ‰ | 29,1 | |
| R1 | Tain 1 | | | 61,8 µl (61,754) | 27,0 ‰ | 26,9 | |
| R2 | Tain 2 | | | 68,6 µl (68,554) | 30,4 ‰ | 30,1 | |
| DDG1 | Domaine des Gardes 1 | | | 49,4 µl (49,344) | 33,7 ‰ | 30,9 | |
| DDG2 | Domaine des Gardes 2 | | | 43,8 µl (43,807) | 26,8 ‰ | 25,9 | |
| PSM | Cognac Prince St Méran | | | 10,8 µl (10,861) | 92,5 ‰ | | High Eth = Low [Zn] = High ‰ |

Dilution des échantillons de vins (sans minéralisation) pour l'analyse des rapports isotopiques de Zn

| Echantillons (50 ppb dans 10 ml) | Vin (ml) | HNO$_3$ 0,5 M+ In 1 ppb | Concentration mesurée |
|---|---|---|---|
| **CM** | 0,899 | 9,101 | ~ 51 ppb |
| **CBN** | 0,966 | 9,034 | ~ 56 ppb |
| **R1** | 0,486 | 9,514 | ~ 58 ppb |
| **R2** | 0,438 | 9,562 | ~ 53 ppb |
| **DDG1** | 0,609 | 9,391 | ~ 53 ppb |
| **DDG2** | 0,686 | 9,314 | ~ 52 ppb |
| **PSM** | 2,765 | 7,235 | Ethanol interférence |
| HNO3 0,5 M + In 1ppb est la combinaison d'une préparation acide composée de HNO3 + d'un acide inorganique In (de type HCL en très faible quantité 1ppb) | | | |

**Résultats** :

Les échantillons marqués ont été comparés à leurs échantillons de référence (vins non marqués) suivant l'équation :

$$\delta_{Zn}^{68} = \left[ \frac{\left( {}^{68}\!/\!{}_{67\,Marked\,wine} \right)}{\left( {}^{68}\!/\!{}_{67\,Reference\,Wine} \right)} - 1 \right] * 1000$$

Les analyses obtenues avec et sans minéralisation ont montré globalement les mêmes résultats. Deux sessions de tests ont été effectuées. Par défaut, les liquides n'ont en principe pas besoin de minéralisation pour être analysés mais, afin de démontrer la viabilité et la cohérence des résultats, nous avons tout de même vérifié après minéralisation des échantillons liquides via une préparation chimique à base d'acides tels que HNO3, HCL, H2O2...) permettant ainsi une solubilité totale des éléments encore présents dans le liquide).

2ème série des tests effectués avec des bouteilles :

Ces analyses ont été effectuées pour tester le mélange d'une goutte (14,8 $\mu$l) de solution marquée dans une bouteille de vin et de Champagne (75 cl). Le marquage isotopique 68Zn a été effectué pour obtenir une variation de 30 ‰ et plus.

| | Test vin rouge | | | Test Champagne | | |
|---|---|---|---|---|---|---|
| | Domaine des Gardes | | | Brut de Noirs, A. Bonnet | | |
| Zn concentrat ion | 1141,4 ng/ml | | | 517,5 ng/ml | | |
| Volume 250 ppm $^{68}$Zn | 14,8 $\mu$l | | | 9,4 $\mu$l | | |
| $^{68}$Zn quantité | 3,516 $\mu$g | | | 2,215 $\mu$g | | |
| Vin (pour dilution 10 ml) | 0,609 ml(50 ppb) | | | 0,966 ml (50 ppb) | | |
| | | | | | | |
| | Echantillonage | $^{68}$Zn/$^{67}$Zn | $\delta^{68}$Zn | Echantillonage | $^{68}$Zn/$^{67}$Zn | $\delta^{68}$Zn |
| Naturel | | 4,8786 | | | 4,8921 | |
| Verre 1 | + 10 min | 4,9373 | 12,03 ‰ | + 15 min | 5,6028 | 145,28 ‰ |
| Verre 2 | + 10 min | 4,9348 | 11,52 ‰ | + 15 min | 5,0626 | 34,85 ‰ |
| Verre 3 | + 10 min | 4,9334 | 11,23 ‰ | + 15 min | 5,0098 | 24,06 ‰ |
| Verre 4 | + 2 h | 5,0424 | 33,58 ‰ | + 1 h | 4,9838 | 18,74 ‰ |
| Dernier mélange | + 2 h | 5,0606 | 37,31 ‰ | + 1 h | 4,9819 | 18,36 ‰ |

3e série de tests : mesure de l'écart type sur les codes uniques

Les analyses des rapports isotopiques sur ICP-MS peuvent présenter des variations d'écart type sur plusieurs analyses. Pour créer un code unique différenciable, nous avons testé plusieurs codes pour les ratios d'isotopes Zn, DZn (Zn appauvri en un isotope ) et Fe. Ces tests ont été effectués sur 10 ml d'aliquotes du même vin DDG1 pour des variations (dopages) de 3, 5, 8, 10, 12, 15 et 30 ‰.

| $^{68}$Zn traceur | $^{68}$Zn pour 10 ml (0,5 ppm) | $^{68}$Zn pour 10 ml | $^{68}$Zn pour 75 cl | $\delta^{68}$Zn ‰ mesuré | concentrations Zn mesurées |
|---|---|---|---|---|---|
| 0 ‰ | | | | | 46,27 ng/ml (0,41 %) |
| 3 ‰ | 9,4 µl (9,375) | 4,69 ng | 351,57 ng | 2,80 | 45,30 ng/ml (0,48 %) |
| 5 ‰ | 15,6 µl (15,625) | 7,81 ng | 585,96 ng | 4,46 | 45,66 ng/ml (0,60 %) |
| 8 ‰ | 25 µl | 12,50 ng | 937,53 ng | 8,95 | 45,40 ng/ml (0,68 %) |
| 10 ‰ | 31,2 µl (31,251) | 15,63 ng | 1171,91 ng | 9,67 | 45,39 ng/ml (0,72 %) |
| 12 ‰ | 37,6 µl (37,50) | 18,75 ng | 1406,30 ng | 12,36 | 46,01 ng/ml (0,79 %) |
| 15 ‰ | 47,0 µl (46,87) | 23,44 ng | 1757,87 ng | 16,35 | 46,15 ng/ml (0,99 %) |
| 30 ‰ | | 46,88 ng | 3515,73 ng | | |

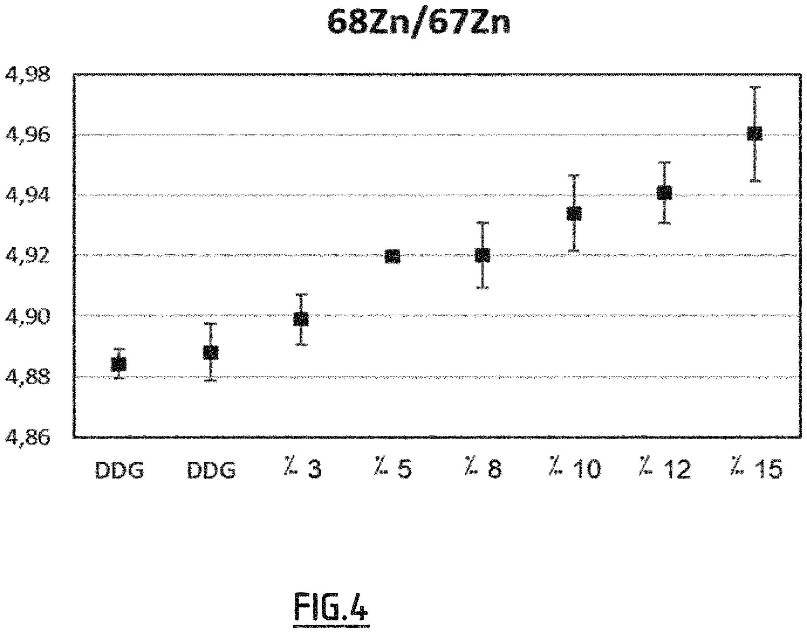

Voir Figure 4.

Comparé aux abondances naturelles de Zn, le marqueur 68ZnCl2 est fortement enrichi en 68Zn et appauvri en isotopes 64, 66, 67 et 70 de Zn.

Sur le rapport 67Zn/66Zn isotopique, le marqueur n'est pas observable). Dans les barres d'erreur, les résultats sont les mêmes.

Sur le rapport 68Zn/67Zn isotopique, les valeurs sont différentes en fonction du marquage 68Zn. Les deux premières valeurs correspondent à l'échantillon de vin DDG1 sans marquage. Dans les barres d'erreur, il sera plus facile de différencier le marquage de 3‰ par rapport à l'échantillon de référence (non marqué) avec un MC-ICP-MS.

Résultats du marquage isotopique 57Fe :

Lors de nos essais, les isotopes analysés sont 54Fe, 56Fe et 57Fe. 58Fe n'a pas été surveillé en raison de l'interférence avec 58Ni. 54Fe est également interféré par le 54Cr.

Sur le rapport 54Fe/56Fe, l'isotope 57Fe n'est pas présent et tous les tests de marquage (5-10-15-20-25-30) peuvent avoir la même valeur dans les erreurs. C'est le cas lorsque la correction (54Cr) est appliquée sur le 54Fe. Sans correction, ces ratios ont des valeurs plus élevées et différentes en raison du signal 54Ni.

La correction n'a aucune incidence sur le ratio 57Fe/56Fe. Comme les rapports isotopiques Zn, la distinction sur le marquage 57Fe est possible pour toutes les variations de marquage de 5‰. Pour un marquage de 30 ‰ et plus, l'erreur sur la concentration de Fe est de 1,5 % de La DSR (Déviation Standard Relative, moyenne des isotopes 54, 56 et 57). Pour l'analyse de la concentration, 56Fe est l'analyse isotopique recommandée, mais 57Fe peut également être analysé.

Voir Figures 5 à 10.

Les deux tests avec le même échantillon DDG1 et la même quantité de marqueur a permis de valider la reproductibilité.

$$\delta^{57}_{Fe} = \left[ \frac{\left(^{57}/_{56\,Marked\,wine}\right)}{\left(^{57}/_{56\,Reference\,Wine}\right)} - 1 \right] * 1000$$

Exemple de marquage et encodage unique du domaine des gardes (1 échantillon de 10ml et une bouteille de 75cl)

| Traceur | $^{57}$Fe pour 10 ml (4 ppm) | $^{57}$Fe pour 10 ml (4 ppm) | $^{57}$Fe pour 10 ml (4 ppm) | $^{57}$Fe pour 10 ml (4 ppm) | $\delta^{57}$Fe (‰) (moyenne) |
|---|---|---|---|---|---|
| 3 ‰ DGG1 | 10,4 µl | 1,245 ng | 93,406 ng | (0,20 %) | 2,98 ± 1.30 |
| 5 ‰ DGG1 | 17,4 µl | 2,076 ng | 155,676 ng | (0,20 %) | 3,19 ± 1.14 |

(suite)

| Traceur | $^{57}$Fe pour 10 ml (4 ppm) | $^{57}$Fe pour 10 ml (4 ppm) | $^{57}$Fe pour 10 ml (4 ppm) | $^{57}$Fe pour 10 ml (4 ppm) | $\delta^{57}$Fe (‰) (moyenne) |
|---|---|---|---|---|---|
| 8 ‰ DGG1 | 27,8 μl | 3,321 ng | 249,082 ng | (0,37 %) | 6,37 ± 0.11 |
| 10 ‰ DGG1 | 34,6 μl | 4,151 ng | 311,352 ng | (0,35 %) | 5,96 ± 4.40 |
| 12 ‰ DGG1 | 41,6 μl | 4,982 ng | 373,622 ng | (0,58 %) | 10,10 ± 0.08 |
| 15 ‰ DGG1 | 52,0 μl | 6,227 ng | 467,028 ng | (0,63 %) | 10,99 ± 0.42 |
| 30 ‰ DGG1 | 103,8 μl | 12,454 ng | 9 ng | (1,50 %) | 26,26 ± 0.10 |

En comparant les valeurs analysées/mesurées en spectrométrie de masse et les données stockées dans la base des codes uniques, nous pouvons déterminer avec précision l'origine de la provenance d'un vin. L'analyse des marqueurs du Zinc enrichi ou appauvri et du Fer nous permettent d'obtenir une précision de l'origine à l'échelle locale tout en minimisant le nombre d'analyses à réaliser.

Concernant les corrections des interférences isotopiques, on peut procéder des deux manières suivantes :

- Choix d'un spectromètre de masse très sensible, par exemple un HR-ICP/MS Triple Quad combiné à l'utilisation de l'hélium.

La capacité multi-élémentaire et les basses limites de détection des spectromètres de masse à couplage inductif (ICP-MS) sont des atouts majeurs pour l'analyse de traces de métaux à l'état de traces. Toutefois, l'existence d'interférences polyatomiques peut gêner ce type de déterminations. L'utilisation d'ICP-MS à cellule de collision/réaction (CCR) permet de supprimer de telles interférences. Des gaz réactifs sont généralement ajoutés mais la mise en oeuvre d'un gaz de collision comme l'hélium offre de nouvelles perspectives pour supprimer simultanément et avec un seul jeu de conditions l'ensemble des interférences dans des matrices complexes et de composition variable.

- Préparation d'une séparation chimique isotopique permettant d'isoler/de retenir uniquement les éléments à analyser. Cette préparation varie en fonction des éléments utilisés.

**Revendications**

1. Méthode permettant d'imposer un code unique, comprenant une signature isotopique, propre à des liquides, de préférence vin ou spiritueux, d'un lieu d'origine ou à des sous-produits de ce liquide,

- on dispose de la signature géographique basale (SGB) du liquide ou de son sous-produit, la SGB comprenant :

(i) les concentrations d'au moins 5, 6, 7, 8, 9, 10, 11, 12 ou la totalité des éléments du groupe (3) Rb, Sr, B Li, Ca, Na, Mg, K, F, P, Cl, As, Pb, Cd,
(ii) les concentrations d'au moins 5, 10, 15, 20, 25, 30, ou la totalité des éléments du groupe (2) : Be, Al, V, Cr, Mn, Fe, Co, Ni, Cu, Zn, Ga, Ge, Se, Y, Zr, Nb, Mo, Rh, Pd, Ag, Sn, Sb, Te, I, Ba, Hf, Ta, W, Re, Ir, Hg, Ti, Si, et
(iii) les concentrations d'isotopes stables d'au moins 1, 2, 3, 4, 5, 6, 7, 8, 9 ou 10 des éléments de ce groupe (2) ;

- la méthode comprenant l'ajout à ce liquide ayant cette SGB, d'une quantité connue d'au moins 1 isotope stable d'au moins 1, 2 ou 3 éléments chimiques du groupe (2), ce grâce à quoi l'on obtient un liquide ayant une signature isotopique déterminée, avec un ratio d'isotope modifié par rapport à la SGB.

2. Méthode selon la revendication 1, dans laquelle la SGB comprend en outre les concentrations des éléments chimiques d'au moins 2, 3, 4 ou les 5 éléments chimiques du groupe (1) : C, O, N, H, S.

3. Méthode selon la revendication 1 ou 2, dans laquelle la SGB comprend des concentrations d'isotopes stables d'au moins 1, 2 ou 3 des éléments du groupe (2') Al, Mn, Fe, Co, Cu, Zn, Mo, Sn, Ti, Si ; et/ou dans laquelle on ajoute au liquide une quantité connue d'au moins 1 isotope stable d'au moins 1, 2 ou 3 des éléments du groupe (2') Al,

Mn, Fe, Co, Cu, Zn, Mo, Sn, Ti, Si.

4. Méthode selon la revendication 3, dans laquelle la SGB comprend les concentrations d'au moins 2 isotopes stables d'au moins 1, 2 ou 3 des éléments Fe, Cu, Zn, Mo, Sn, Ti, Si.

5. Méthode selon la revendication 3, dans laquelle on détermine pour la SGB les concentrations d'au moins 2 isotopes stables d'au moins 1 élément parmi Fe et Zn.

6. Méthode selon la revendication 5, dans laquelle on mesure 56Fe et 57Fe, et/ou 66Zn et 68Zn, et, par ajout d'isotope dans le liquide, on fait varier la concentration de 56Fe et/ou de 57Fe, et/ou de 66Zn et/ou de 68Zn.

7. Méthode selon l'une quelconque des revendications 1 à 6, dans laquelle la SGB comprend les concentrations des éléments Rb, Sr et B ; et/ou les concentrations des éléments Li, Ca, Na, Mg, K, F, P, CI, As, Pb, Cd.

8. Méthode selon l'une quelconque des revendications 1 à 7, dans laquelle le code unique est défini par un calculateur électronique en fonction des codes uniques déjà générés et de la SGB du liquide ou de son sous-produit, ce calculateur électronique comprenant un modèle (M) contenant en mémoire les codes uniques déjà générés.

9. Méthode selon la revendication 8, dans laquelle on mesure la SGB de liquides ou de sous-produits successifs et l'on définit à chaque fois un code unique au liquide ou sous-produit dernièrement analysé, code qui se distingue des codes uniques précédemment définis et enregistrés dans le modèle (M).

10. Méthode d'identification isotopique permettant de relier un liquide ou un sous-produit, de préférence vin ou spiritueux, à un lieu d'origine, la méthode comprenant :

    a- dans un échantillon du liquide ou du sous-produit, la mesure :

    (i) des concentrations d'au moins 5, 6, 7, 8, 9, 10, 11, 12 ou la totalité des éléments du groupe (3) Rb, Sr, B Li, Ca, Na, Mg, K, F, P, CI, As, Pb, Cd,
    (ii) des concentrations d'au moins 5, 10, 15, 20, 25, 30, ou la totalité des éléments du groupe (2) : Be, AI, V, Cr, Mn, Fe, Co, Ni, Cu, Zn, Ga, Ge, Se, Y, Zr, Nb, Mo, Rh, Pd, Ag, Sn, Sb, Te, I, Ba, Hf, Ta, W, Re, Ir, Hg, Ti, Si, et
    (iii) les concentrations d'isotopes stables d'au moins 2, 3, 4, 5, 6, 7, 8, 9 ou 10 des éléments de ce groupe (2) ;

    obtention d'un profil de concentrations d'éléments et d'isotopes stables;
    b- la comparaison de ce profil avec des profils enregistrés dans un modèle prédéfini (M) contenant en mémoire des profils sous forme de codes uniques chacun propre à un type de liquide ou sous-produit d'un lieu d'origine, chaque code unique ayant été préalablement généré par le modèle (M) avec une variation d'isotopes d'éléments en appliquant la méthode selon l'une quelconque des revendications 1 à 9,
    c- la conclusion que le liquide ou le sous-produit dérivé à identifier a un profil sensiblement égal à un code enregistré et donc indication d'un lieu d'origine, si, à l'issue de la comparaison, le profil correspond à un profil enregistré et, dans le cas contraire, la conclusion que le liquide ou le sous-produit dérivé ne provient d'aucun lieu d'origine dont le code est enregistré dans le modèle, ou que le liquide a été falsifié.

11. Méthode selon la revendication 10, dans laquelle on mesure en outre dans un échantillon du liquide ou du sous-produit, les concentrations d'au moins 2, 3, 4, ou les 5 éléments chimiques du groupe (1) : C, O, N, H, S.

12. Méthode selon la revendication 10 ou 11, dans laquelle le modèle M comprend des codes uniques générés avec des SGB comprenant des concentrations d'isotopes stables d'au moins 1, 2 ou 3 des éléments du groupe (2') AI, Mn, Fe, Co, Cu, Zn, Mo, Sn, Ti, Si, et avec un ajout au liquide d'une quantité connue d'au moins 1 isotope stable d'au moins 1, 2 ou 3 des éléments du groupe (2') AI, Mn, Fe, Co, Cu, Zn, Mo, Sn, Ti, Si.

13. Méthode selon la revendication 12, dans laquelle la SGB comprenait les concentrations d'au moins 2 isotopes stables d'au moins 1, 2 ou 3 des éléments Fe, Cu, Zn, Mo, Sn, Ti, Si, de préférence les concentrations d'au moins 2 isotopes stables d'au moins 1 élément parmi Fe et Zn.

14. Méthode selon la revendication 13, dans laquelle on a mesuré 56Fe et 57Fe, et/ou 66Zn et 68Zn, et, par ajout d'isotope dans le liquide, on a fait varier la concentration de 56Fe et/ou de 57Fe, et/ou de 66Zn et/ou de 68Zn.

**15.** Méthode selon l'une quelconque des revendications 10 à 14, dans laquelle la SGB comprenait les concentrations des éléments Rb, Sr et B ; et/ou les concentrations des éléments Li, Ca, Na, Mg, K, F, P, CI, As, Pb, Cd.

**16.** Méthode selon l'une quelconque des revendications 10 à 15, comprenant l'utilisation d'un calculateur électronique dans lequel sont enregistrés les codes uniques propres à d'autres liquides d'au moins un autre lieu d'origine, et/ou dans laquelle est enregistré la SGB du liquide d'un lieu de d'origine pour comparaison avec les profils enregistrés.

**17.** Calculateur électronique (2) utilisable pour la mise en oeuvre de la méthode d'identification isotopique selon l'une quelconque des revendications 10 à 16, comportant une unité logique (3) programmable et un support d'enregistrement d'informations (4) contenant des instructions logicielles adaptées pour, lorsqu'elles sont exécutées par l'unité logique (3), mettre en oeuvre des étapes de comparaison d'un profil de concentrations ou de ratios d'éléments et d'isotopes stables, d'un échantillon issu d'un liquide ou sous-produit, sous forme de concentrations (C2) ou de ratios (R2) , avec des profils enregistrés sous forme de codes uniques chacun propre à un lieu d'origine, et de détermination si le liquide ou sous-produit a un profil sensiblement égal à un code enregistré et donc indication du lieu d'origine, ou que le liquide ou le sous-produit ne provient d'aucun lieu d'origine dont le code est enregistré dans le modèle, ou que le liquide a été falsifié, le support d'enregistrement d'informations (4) comprenant en mémoire les codes uniques d'au moins un vin ou spiritueux, ce code unique étant représentatif d'une appellation, d'un domaine et/ou d'un millésime et comprenant, pour chaque vin ou spiritueux, les concentrations d'au moins 5, 6, 7, 8, 9, 10, 11, 12 ou la totalité des éléments du groupe (3) Rb, Sr, B Li, Ca, Na, Mg, K, F, P, CI, As, Pb, Cd, et d'au moins 5, 10, 15, 20, 25, 30, ou la totalité des éléments du groupe (2) : Be, Al, V, Cr, Mn, Fe, Co, Ni, Cu, Zn, Ga, Ge, Se, Y, Zr, Nb, Mo, Rh, Pd, Ag, Sn, Sb, Te, I, Ba, Hf, Ta, W, Re, Ir, Hg, Ti, Si, et des concentrations ou des ratios d'isotopes d'au moins 1, 2 ou 3 des éléments du groupe (2') Al, Mn, Fe, Co, Cu, Zn, Mo, Sn, Ti, Si.

**Patentansprüche**

**1.** Verfahren, das es ermöglicht, eine eindeutigen Code aufzuerlegen, umfassend eine Isotopensignatur, die für Flüssigkeiten, vorzugsweise Wein oder Spirituosen, eines Herkunftsorts oder für Nebenprodukte dieser Flüssigkeit spezifisch ist,

- Verfügen über die basale geographische Signatur (SGB) der Flüssigkeit oder ihres Nebenprodukts, die SGB umfassend:

(i) die Konzentrationen von mindestens 5, 6, 7, 8, 9, 10, 11, 12 oder allen Elementen der Gruppe (3) Rb, Sr, B Li, Ca, Na, Mg, K, F, P, CI, As, Pb, Cd,
(ii) die Konzentrationen von mindestens 5, 10, 15, 20, 25, 30 oder allen Elementen der Gruppe (2): Be, Al, V, Cr, Mn, Fe, Co, Ni, Cu, Zn, Ga, Ge, Se, Y, Zr, Nb, Mo, Rh, Pd, Ag, Sn, Sb, Te, I, Ba, Hf, Ta, W, Re, Ir, Hg, Ti, Si, und
(iii) Konzentrationen von stabilen Isotopen von mindestens 1, 2, 3, 4, 5, 6, 7, 8, 9 oder 10 der Elemente in dieser Gruppe (2);

- das Verfahren umfassend das Hinzufügen einer bekannten Menge von mindestens einem stabilen Isotop von mindestens 1, 2 oder 3 chemischen Elementen der Gruppe (2), wodurch eine Flüssigkeit erlangt wird, die eine bestimmte Isotopensignatur mit einem veränderten Isotopenverhältnis in Bezug auf den SGB aufweist.

**2.** Verfahren nach Anspruch 1, wobei die SGB zusätzlich die Konzentrationen der chemischen Elemente von mindestens 2, 3, 4 oder allen 5 chemischen Elementen der Gruppe (1) umfasst: C, O, N, H, S.

**3.** Verfahren nach Anspruch 1 oder 2, wobei die SGB Konzentrationen von stabilen Isotopen von mindestens 1, 2 oder 3 der Elemente der Gruppe (2') Al, Mn, Fe, Co, Cu, Zn, Mo, Sn, Ti, Si umfasst; und/oder wobei zu der Flüssigkeit eine bekannte Menge von mindestens einem stabilem Isotop von mindestens 1, 2 oder 3 der Elemente aus der Gruppe (2') Al, Mn, Fe, Co, Cu, Zn, Mo, Sn, Ti, Si hinzugefügt wird.

**4.** Verfahren nach Anspruch 3, wobei die SGB die Konzentrationen von mindestens 2 stabilen Isotopen von mindestens 1, 2 oder 3 der Elemente Fe, Cu, Zn, Mo, Sn, Ti, Si umfasst.

**5.** Verfahren nach Anspruch 3, wobei für die SGB die Konzentrationen von mindestens 2 stabilen Isotopen von mindestens 1 Element aus Fe und Zn bestimmt werden.

6. Verfahren nach Anspruch 5, wobei 56Fe und 57Fe und/oder 66Zn und 68Zn gemessen werden und durch Hinzufügen des Isotops in die Flüssigkeit die Konzentration von 56Fe und/oder 57Fe und/oder 66Zn und/oder 68Zn variiert wird.

7. Verfahren nach einem der Ansprüche 1 bis 6, wobei die SGB die Konzentrationen der Elemente Rb, Sr und B; und/oder die Konzentrationen der Elemente Li, Ca, Na, Mg, K, F, P, Cl, As, Pb, Cd umfasst.

8. Verfahren nach einem der Ansprüche 1 bis 7, wobei der eindeutige Code von einem elektronischen Rechner abhängig von den bereits erzeugten eindeutigen Codes und der SGB der Flüssigkeit oder ihres Nebenprodukts definiert wird, der elektronische Rechner umfassend ein Modell (M), die in Speicher die bereits erzeugten eindeutigen Codes enthält.

9. Verfahren nach Anspruch 8, bei dem die SGB von Flüssigkeiten oder aufeinanderfolgenden Nebenprodukten gemessen wird und jedes Mal ein eindeutiger Code für die zuletzt analysierte Flüssigkeit oder das zuletzt analysierte Nebenprodukt definiert wird, wobei sich der Code von den zuvor definierten und in dem Modell (M) gespeicherten eindeutigen Codes unterscheidet.

10. Isotopenidentifikationsverfahren, das es ermöglicht, eine Flüssigkeit oder ein Nebenprodukt, vorzugsweise Wein oder Spirituosen, mit einem Herkunftsort in Verbindung zu bringen, das Verfahren umfassend:

   a) in einer Probe der Flüssigkeit oder des Nebenprodukts das Messen:

   (i) von Konzentrationen von mindestens 5, 6, 7, 8, 9, 10, 11, 12 oder allen Elementen der Gruppe (3) Rb, Sr, B Li, Ca, Na, Mg, K, F, P, Cl, As, Pb, Cd,
   (ii) von Konzentrationen von mindestens 5, 10, 15, 20, 25, 30 oder allen Elementen der Gruppe (2): Be, Al, V, Cr, Mn, Fe, Co, Ni, Cu, Zn, Ga, Ge, Se, Y, Zr, Nb, Mo, Rh, Pd, Ag, Sn, Sb, Te, I, Ba, Hf, Ta, W, Re, Ir, Hg, Ti, Si, und
   (iii) die Konzentrationen von stabilen Isotopen von mindestens 2, 3, 4, 5, 6, 7, 8, 9 oder 10 der Elemente dieser Gruppe (2);

   Erlangen eines Konzentrationsprofils stabiler Elemente und Isotope;
   b) Vergleichen dieses Profils mit Profilen, die in einem vordefinierten Modell (M) gespeichert sind, das Profile in Form von eindeutigen Codes speichert, die jeweils für eine Art von Flüssigkeit oder Nebenprodukt eines Herkunftsorts spezifisch sind, wobei jeder eindeutige Code zuvor durch das Modell (M) mit einer Variation von Element-Isotopen unter Anwendung des Verfahrens nach einem der Ansprüche 1 bis 9 erzeugt wurde,
   c) die Schlussfolgerung, dass die zu identifizierende Flüssigkeit oder das zu identifizierende Nebenprodukt ein Profil aufweist, das im Wesentlichen einem registrierten Code und damit einem Hinweis auf einen Herkunftsort entspricht, wenn das Profil nach dem Vergleich mit einem registrierten Profil entspricht, und andernfalls die Schlussfolgerung, dass die Flüssigkeit oder das daraus abgeleitete Nebenprodukt aus keinem Herkunftsort stammt, dessen Code in dem Modell registriert ist, oder dass die Flüssigkeit gefälscht wurde.

11. Verfahren nach Anspruch 10, wobei zusätzlich in einer Probe der Flüssigkeit oder des Nebenprodukts die Konzentrationen von mindestens 2, 3, 4 oder allen 5 chemischen Elementen der Gruppe (1) gemessen werden: C, O, N, H, S.

12. Verfahren nach Anspruch 10 oder 11, wobei das Modell M eindeutige Codes umfasst, die mit SGB erzeugt werden, umfassend Konzentrationen stabiler isotope von mindestens 1, 2 oder 3 der Elemente der Gruppe (2') Al, Mn, Fe, Co, Cu, Zn, Mo, Sn, Ti, Si, und mit einem Hinzufügen einer bekannten Menge von mindestens 1 stabilen Isotop von mindestens 1, 2 oder 3 der Elemente der Gruppe (2') Al, Mn, Fe, Co, Cu, Zn, Mo, Sn, Ti, Si zu der Flüssigkeit.

13. Verfahren nach Anspruch 12, wobei die SGB die Konzentrationen von mindestens 2 stabilen Isotopen von mindestens 1, 2 oder 3 der Elemente Fe, Cu, Zn, Mo, Sn, Ti, Si, vorzugsweise die Konzentrationen von mindestens 2 stabilen Isotopen von mindestens 1 Element aus Fe und Zn, umfasst.

14. Verfahren nach Anspruch 13, wobei 56Fe und 57Fe und/oder 66Zn und 68Zn gemessen werden und durch Hinzufügen des Isotops in die Flüssigkeit die Konzentration von 56Fe und/oder 57Fe und/oder 66Zn und/oder 68Zn variiert wird.

15. Verfahren nach einem der Ansprüche 10 bis 14, wobei die SGB die Konzentrationen der Elemente Rb, Sr und B; und/oder die Konzentrationen der Elemente Li, Ca, Na, Mg, K, F, P, Cl, As, Pb, Cd umfasst.

**16.** Verfahren nach einem der Ansprüche 10 bis 15, umfassend die Verwendung eines elektronischen Rechners, in dem eindeutige Codes gespeichert sind, die für andere Flüssigkeiten aus mindestens einem anderen Herkunftsort typisch sind, und/oder in dem die SGB der Flüssigkeit aus einem Herkunftsort zum Vergleich mit den gespeicherten Profilen gespeichert ist.

**17.** Elektronischer Rechner (2), der für die Durchführung des Isotopenidentifikationsverfahrens nach einem der Ansprüche 10 bis 16 verwendbar ist, umfassend eine programmierbaren Logikeinheit (3) und ein Informationsaufzeichnungsmedium (4), das Softwareanweisungen enthält, die angepasst sind, wenn sie von der Logikeinheit (3) ausgeführt werden, zum Durchführen von Schritten zum Vergleichen eines Profils von Konzentrationen oder Verhältnissen von Elementen und stabilen Isotopen einer Probe aus einer Flüssigkeit oder einem Nebenprodukt in Form von Konzentrationen (C2) oder Verhältnissen (R2) mit Profilen, die als eindeutige Codes gespeichert sind, die jeweils für einen Herkunftsort spezifisch sind, und Bestimmen, ob die Flüssigkeit oder das Nebenprodukt ein Profil aufweist, das im Wesentlichen einem registrierten Code und damit einer Angabe des Ursprungsorts entspricht, oder ob die Flüssigkeit oder das Nebenprodukt von keinem Herkunftsort stammt, dessen Code in dem Muster registriert ist, oder ob die Flüssigkeit gefälscht wurde, das Informationsaufzeichnungsmedium (4) umfassend im Speicher die eindeutigen Codes von mindestens einem Wein oder einer Spirituose, wobei dieser eindeutige Code für eine Bezeichnung, ein Weingut und/oder einen Jahrgang repräsentativ ist, und umfassend für jeden Wein oder jede Spirituose, die Konzentrationen von mindestens 5, 6, 7, 8, 9, 10, 11, 12 oder allen Elementen der Gruppe (3) Rb, Sr, B Li, Ca, Na, Mg, K, F, P, Cl, As, Pb, Cd und von mindestens 5, 10, 15, 20, 25, 30 oder allen Elementen der Gruppe (2): Be, Al, V, Cr, Mn, Fe, Co, Ni, Cu, Zn, Ga, Ge, Se, Y, Zr, Nb, Mo, Rh, Pd, Ag, Sn, Sb, Te, I, Ba, Hf, Ta, W, Re, Ir, Hg, Ti, Si, und Isotopenkonzentrationen oder -verhältnisse von mindestens 1, 2 oder 3 der Elemente der Gruppe (2') Al, Mn, Fe, Co, Cu, Zn, Mo, Sn, Ti, Si.

**Claims**

**1.** Method allowing marking a unique code, comprising an isotropic signature, specific to liquids, preferably wines or spirits, of a place of origin or to by-products of this liquid,

- providing the basic geographic signature (BGS) of the liquid or its by-product, the SGB comprising:

(i) the concentrations of at least 5, 6, 7, 8, 9, 10, 11, 12 or the entirety of the elements in group (3): Rb, Sr, B Li, Ca, Na, Mg, K, F, P, Cl, As, Pb, Cd,
(ii) the concentrations of at least 5, 10, 15, 20, 25, 30, or the entirety of the elements in group (2): Be, Al, V, Cr, Mn, Fe, Co, Ni, Cu, Zn, Ga, Ge, Se, Y, Zr, Nb, Mo, Rh, Pd, Ag, Sn, Sb, Te, I, Ba, Hf, Ta, W, Re, Ir, Hg, Ti, Si, and
(iii) the concentrations of stable isotopes of at least 1, 2, 3, 4, 5, 6, 7, 8, 9 or 10 of the elements in this group (2);

- the method comprising the addition to this liquid having this BGS of a known quantity of at least 1 stable isotope of at least 1, 2 or 3 chemical elements of group (2), by means of which a liquid is obtained having a determined isotopic signature, with a modified isotope ratio compared with the BGS.

**2.** The method according to claim 1, wherein the BGS also comprises the concentrations of chemical elements of at least 2, 3, 4 or the 5 chemical elements in group (1): C, O, N, H, S.

**3.** The method according to claim 1 or 2, wherein the BGS comprises concentrations of stable isotopes of at least 1, 2 or 3 of the elements in group (2') Al, Mn, Fe, Co, Cu, Zn, Mo, Sn, Ti, Si; and/ or the addition is made to the liquid of a known quantity of at least 1 stable isotope of at least 1, 2 or 3 of the elements in group (2') Al, Mn, Fe, Co, Cu, Zn, Mo, Sn, Ti, Si.

**4.** The method according to claim 3, wherein the BGS comprises the concentrations of at least 2 stable isotopes of at least 1, 2 or 3 of the elements Fe, Cu, Zn, Mo, Sn, Ti, Si.

**5.** The method according to claim 3, wherein, for the BGS, the concentrations are determined of at least 2 stable isotopes of at least 1 element from among Fe and Zn.

**6.** The method according to claim 5, wherein $^{56}Fe$ and $^{57}Fe$, and/or $^{66}Zn$ and $^{68}Zn$ are measured and, via addition of isotope to the liquid, the concentration is varied of $^{56}Fe$ and/or of $^{57}Fe$, and/or of $^{66}Zn$ and/or of $^{68}Zn$.

7. The method according to any of claims 1 to 6, wherein the BGS comprises the concentrations of the elements Rb, Sr and B; and/or the concentrations of the elements Li, Ca, Na, Mg, K, F, P, Cl, As, Pb, Cd.

8. The method according to any of claims 1 to 7, wherein the unique code is defined by an electronic computer as a function of the unique codes already generated and of the BGS of the liquid or its by-product, this electronic computer comprising a model (M) having in memory the unique codes already generated.

9. The method according to claim 8, wherein the BGS is measured of liquids or successive by-products, and each time a unique code is defined for the liquid or last-analysed by-product, this code differing from the unique codes previously defined and recorded in the model (M).

10. An isotopic identification method allowing a liquid or a by-product, preferably a wine or spirit, to be linked with a place of origin, the method comprising:

   a- in a sample of the liquid or the by-product, measuring:

   (i) the concentrations of at least 5, 6, 7, 8, 9, 10, 11, 12 or the entirety of the elements in group (3) Rb, Sr, B Li, Ca, Na, Mg, K, F, P, Cl, As, Pb, Cd,
   (ii) the concentrations of at least 5, 10, 15, 20, 25, 30, or the entirety of the elements in group (2): Be, Al, V, Cr, Mn, Fe, Co, Ni, Cu, Zn, Ga, Ge, Se, Y, Zr, Nb, Mo, Rh, Pd, Ag, Sn, Sb, Te, I, Ba, Hf, Ta, W, Re, Ir, Hg, Ti, Si, and
   (iii) the concentrations of stable isotopes of at least 2, 3, 4, 5, 6, 7, 8, 9 or 10 of the elements in this group (2);

   to obtain a profile of concentrations of elements and stable isotopes;
   b- comparing this profile with profiles recorded in a predefined model (M) having in memory profiles in memory in the form of unique codes each specific to a type of liquid or by-product from a place of origin, each unique code having been previously generated by the model (M) with a variation in isotopes of elements by applying the method according to any one of claims 1 to 9;
   c- concluding that the liquid or the derivative by-product to be identified has a profile substantially equal to a recorded code and hence indicating a place of origin, if after comparison the profile corresponds to a recorded profile, and on the contrary concluding that the liquid or derivative by-product does not come from any place of origin having a code recorded in the model, or that the liquid has been adulterated.

11. The method according to claim 10, wherein in a sample of the liquid or by-product, the concentrations are also measured of at least 2, 3, 4, or the 5 chemical elements in group (1): C, O, N, H, S.

12. The method according to claim 10 or 11, wherein the model M comprises unique codes generated with BGS comprising concentrations of stable isotopes of at least 1, 2 or 3 of the elements in group (2') Al, Mn, Fe, Co, Cu, Zn, Mo, Sn, Ti, Si, and the addition is made to the liquid of a known quantity of at least 1 stable isotope of at least 1, 2 or 3 of the elements in group (2') Al, Mn, Fe, Co, Cu, Zn, Mo, Sn, Ti, Si.

13. The method according to claim 12, wherein the BGS comprisesd the concentrations of at least 2 stable isotopes of at least 1, 2 or 3 of the elements Fe, Cu, Zn, Mo, Sn, Ti, Si, preferably the concentration au at least 2 stable isotopes of at least 1 element from among Fe and Zn.

14. The method according to claim 13, wherein $^{56}Fe$ and $^{57}Fe$, and/or $^{66}Zn$ and $^{68}Zn$ are measured, and, via isotope addition to the liquid, the concentration is varied of $^{56}Fe$ and/or of $^{57}Fe$, and/or of $^{66}Zn$ and/or of $^{68}Zn$.

15. The method according to any of claims 10 to 14, wherein the BGS comprised the concentrations of the elements Rb, Sr and B; and/or the concentrations of the elements Li, Ca, Na, Mg, K, F, P, Cl, As, Pb, Cd.

16. The method according to any of claims 10 to 15, comprising the use of an electronic computer in which there are recorded the unique codes specific to other liquids from at least one other place of origin, and/or in which the BGS is recorded of the liquid from a place of origin for comparison with the recorded profiles.

17. An electronic computer (2) that can be used to implement the isotopic identification method according to any of claims 10 to 16, comprising a programmable logic unit (3) and data recording medium (4) containing software instructions adapted, when executed by the logic unit (3), for implementing steps to compare a profile of concentrations or ratios of elements and stable isotopes in a sample of a liquid or by-product, in the form of concentrations (C2) or ratios (R2), with profiles recorded in the form of unique codes, each one being specific to a place of origin, and to determine whether the liquid or by-product has a profile substantially equal to a recorded code and hence indicating a place of origin, or whether the liquid or by-product does not come from any place of origin having the code recorded in the model, or that the liquid has been adulterated; the data recording medium (4) having in memory the unique codes of at least one wine or spirit, this unique code representing a label of origin, a vineyard and/or a vintage and, for each wine or spirit, comprising the concentrations of at least 5, 6, 7, 8, 9, 10, 11, 12 or the entirety of the elements in group (3) Rb, Sr, B Li, Ca, Na, Mg, K, F, P, Cl, As, Pb, Cd, and at least 5, 10, 15, 20, 25, 30, or the entirety of the elements in group (2) Be, Al, V, Cr, Mn, Fe, Co, Ni, Cu, Zn, Ga, Ge, Se, Y, Zr, Nb, Mo, Rh, Pd, Ag, Sn, Sb, Te, I, Ba, Hf, Ta, W, Re, Ir, Hg, Ti, Si, and concentrations or ratios of isotopes of at least 1, 2 or 3 of the elements in group (2') Al, Mn, Fe, Co, Cu, Zn, Mo, Sn, Ti, Si.

FIG.1

## FIG.2

FIG.3

**68Zn/67Zn**

FIG.4

EP 3 918 320 B1

FIG.5

FIG.6

## 57Fe/56Fe

**FIG.7**

## 54Fe/56Fe

**FIG.8**

**57Fe/54Fe**

FIG.9

**57Fe/56Fe**

FIG.10

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2012323809 A1